(19) Europäisches Patentamt
European Patent Office
Office européen des brevets

(11) Veröffentlichungsnummer: **0 311 955 B1**

(12) # EUROPÄISCHE PATENTSCHRIFT

(45) Veröffentlichungstag der Patentschrift: **21.04.93**

(51) Int. Cl.$^5$: **C07D 319/02**, A61K 31/335, C07C 409/06

(21) Anmeldenummer: **88116791.0**

(22) Anmeldetag: **10.10.88**

(54) **Bicyclische Peroxide.**

(30) Priorität: **15.10.87 CH 4032/87**
**08.07.88 CH 2612/88**

(43) Veröffentlichungstag der Anmeldung:
**19.04.89 Patentblatt 89/16**

(45) Bekanntmachung des Hinweises auf die Patenterteilung:
**21.04.93 Patentblatt 93/16**

(84) Benannte Vertragsstaaten:
**AT BE CH DE ES FR GB GR IT LI LU NL SE**

(56) Entgegenhaltungen:

**Keine Entgegenhaltungen.**

(73) Patentinhaber: **F. HOFFMANN-LA ROCHE AG**
**Postfach 3255**
**CH-4002 Basel(CH)**

(72) Erfinder: **Hofheinz, Werner, Dr.**
**Talmattweg 7**
**CH-4103 Bottmingen(CH)**
Erfinder: **Schmid, Gérard, Dr.**
**Mittler Feldweg 6**
**CH-4468 Kienberg(CH)**
Erfinder: **Stohler, Harro, Dr.**
**Im Katzenwadel 12**
**CH-4103 Binningen(CH)**

(74) Vertreter: **Lederer, Franz, Dr. et al**
**Patentanwalt Dr. Franz Lederer Lucile-Grahn-Strasse 22**
**W-8000 München 80 (DE)**

EP 0 311 955 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft bicyclische Peroxide der allgemeinen Formel

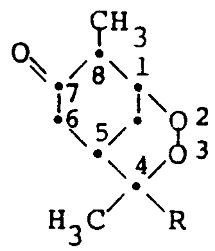

I

worin R eine gesättigte oder partiell ungesättigte Kohlenwasserstoffgruppe mit bis zu 15 Kohlenstoffatomen oder eine über eine Alkyl- oder Alkenylgruppe mit bis zu 7 Kohlenstoffatomen gebundene Aryl-, Heteroaryl-, Arylcarbonyl- oder Heteroarylcarbonylgruppe bedeutet,
wobei Aryl und Heteroaryl in den zuvor genannten Gruppen unsubstituiert oder mit bis zu 5 gleichen oder verschiedenen Substituenten ausgewählt aus Alkyl oder Alkoxy mit jeweils höchstens 7 Kohlenstoffatonen, Halogen, Trifluormethyl, Phenyl und Cyano substituiert sind.

Die neuen Verbindungen der obigen Formel I besitzen wertvolle pharmakologische Eigenschaften. Sie zeigen insbesondere eine ausgeprägte Wirkung gegen die Erreger der Malaria und können zur Verhütung und Bekämpfung der Malaria verwendet werden.

Gegenstand der vorliegenden Erfindung sind: Die obigen Verbindung der Formel I als solche und zur Anwendung als therapeutische Wirkstoffe; ein Verfahren und Zwischenprodukte zu deren Herstellung, Arzneimittel auf der Basis dieser neuen Stoffe; sowie die Verwendung der neuen Verbindungen der Formel I zur Herstellung von Arzneimitteln, welche sich für die Verhütung und Bekämpfung der Malaria eignen.

Der weiter unten verwendete Ausdruck "nieder" bezeichnet Reste und Verbindungen mit höchstens 7, vorzugsweise höchstens 4 Kohlenstoffatomen. Der Ausdruck "Alkyl" für sich allein genommen oder in Zusammensetzungen, wie "Alkylgruppe" und "Alkoxy", bezeichnet geradkettige oder verzweigte, gesättigte Kohlenwasserstoffreste, wie Methyl, Aethyl, n-Propyl, i-Propyl, n-Butyl, s-Butyl, i-Butyl, i-Pentyl, n-Pentyl, n-Hexyl, n-Heptyl, n-Octyl, n-Nonyl, n-Decyl, n-Undecyl, n-Dodecyl, n-Tridecyl, n-Tetradecyl und n-Pentadecyl. Der Ausdruck "Alkenyl" bezeichnet geradkettige oder verzweigte Kohlenwasserstoffreste, welche mindestens eine olefinische Doppelbindung enthalten, wie Vinyl, Allyl, 1-Propenyl, 1-Butenyl, 1-Pentenyl, 1-Hexenyl, 1-Heptenyl, 1-Octenyl, 1-Nonenyl, 1-Decenyl, 1-Undecenyl, 1-Dodecenyl, 1-Tetradecenyl und 4,8-Dimethyl-1,3,7-nonatrienyl.

Der Ausdruck "gesättigte oder partiell ungesättigte Kohlenwasserstoffgruppe" bezeichnet offenkettige und cyclische Gruppen und Kombinationen davon. Beispiele für gesättigte und partiell ungesättigte Kohlenwasserstoffgruppen sind: Alkyl- und Alkenylgruppen, wie sie weiter oben definiert sind; mono-, bi- und tricyclische Alkylgruppen, welche gegebenenfalls über eine Alkyl- oder Alkenylgruppe gebunden sind, wie 3-(1-Adamantyl)propenyl, Cyclopropylmethyl und 2-Cyclohexyläthyl.

Der Ausdruck "Aryl" bezeichnet carbocyclische aromatische Gruppen, vorzugsweise mono- oder bicyclische Gruppen, d.h. Phenyl- und Naphthylgruppen, insbesondere Phenylgruppen. Diese Gruppen sind unsubstituiert oder mit bis zu 5 Substituenten ausgewählt aus niederem Alkyl, niederem Alkoxy, Halogen, Trifluormethyl, Phenyl und Cyano substituiert, wobei die Substituenten gleich oder verschieden sein können. Sie sind dabei vorzugsweise durch Halogen, insbesondere Fluor oder Chlor, und/oder Trifluormethyl mono-, di- oder pentasubstituiert. Als Beispiele für derartige Gruppen seien erwähnt: Phenyl, 4-Fluorphenyl, Pentafluorphenyl, 4-Cyanophenyl, 2-Chlor-4-cyanophenyl, 3,5-Dicyanophenyl, 2-Chlor-4-(trifluormethyl)phenyl, 4-Methoxyphenyl, 4-Methylphenyl, 4-(t-Butyl)phenyl, 4-Chlorphenyl, 2,4-Dichlorphenyl, 3,4-Dichlorphenyl, 3-(Trifluormethyl)phenyl, 4-(Trifluormethyl)phenyl, 3,5- und 2,4-bis(Trifluormethyl)-phenyl, 4-Biphenyl, 1-Naphthyl und 4-Brom-1-naphthyl.

Der Ausdruck "Heteroaryl" bezeichnet heterocyclische aromatische Gruppen, vorzugsweise mono- oder bicyclische Gruppen. Im Falle der bicyclischen Gruppen ist einer der aromatischen Ringe vorzugsweise carbocyclisch. Als Heteroatome kommen vorzugsweise 1, 2 oder 3 Stickstoffatome in Frage. Bevorzugte Gruppen sind Pyridyl-, Pyrimidyl- und insbesondere Chinolinylgruppen. Diese Gruppen sind unsubstituiert oder mit bis zu 5 Substituenten ausgewählt aus niederem Alkyl, niederem Alkoxy, Halogen, Trifluormethyl, Phenyl und Cyano substituiert, wobei die Substituenten gleich oder verschieden sein können. Sie sind dabei vorzugsweise substituiert, und zwar vorzugsweise mono-, di- oder trisubstituiert durch Halogen,

insbesondere Chlor oder Fluor, und/oder Trifluormethyl. Als Beispiele für derartige Gruppen seien erwähnt: 2,7-Bis(trifluormethyl)-4-chinolinyl, 2,8-Bis(trifluormethyl)-4-chinolinyl und 6,8-Dichlor-2-(trifluormethyl)-4-chinolinyl.

Der Ausdruck "Halogen" bezeichnet die vier Formen Fluor, Chlor, Brom und Jod.

Die Verbindungen der Formel I besitzen, sofern R eine von Methyl verschiedene Bedeutung hat, vier asymmetrisch substituierte Kohlenstoffatome. Falls R Methyl bedeutet, besitzen die entsprechenden Verbindungen der Formel I drei asymmetrisch substituierte Kohlenstoffatome. Die vorliegende Erfindung umfasst alle möglichen Stereoisomeren, insbesondere die (1R,4R,5S,8R)-, (1R,4S,5S,8R)-, (1S,4R,5R,8S)- und die (1S,4S,5R,8S)-Isomeren und Mischungen davon.

In einer speziellen Ausführungsform betrifft die vorliegende Erfindung Verbindungen der Formel I, worin R eine gesättigte, vorzugsweise offenkettige und geradkettige Kohlenwasserstoffgruppe mit 8-12 Kohlenstoffatomen bedeutet.

Im Rahmen dieses Aspektes werden die nachfolgend aufgeführten Verbindungen besonders bevorzugt:

4,8-Dimethyl-4-octyl-2,3-dioxabicyclo[3.3.1]nonan-7-on,
4,8-Dimethyl-4-nonyl-2,3-dioxabicyclo[3.3.1]nonan-7-on,
4-Decyl-4,8-dimethyl-2,3-dioxabicyclo[3.3.1]nonan-7-on,
4,8-Dimethyl-4-undecyl-2,3-dioxabicyclo[3.3.1]nonan-7-on und
4-Dodecyl-4,8-dimethyl-2,3-dioxabicyclo[3.3.1]nonan-7-on.

In einer weiteren speziellen Ausführungsform betrifft die vorliegende Erfindung Verbindungen der Formel I, worin R eine über eine Alkenylgruppe mit bis zu 7 Kohlenstoffatomen gebundene Aryl- oder Heteroarylgruppe bedeutet. Die Arylgruppe ist dabei vorzugsweise eine gegebenenfalls durch Halogen, insbesondere Fluor oder Chlor, und/oder Trifluormethyl mono-, di- oder pentasubstituierte Phenylgruppe, und die Heteroarylgruppe ist vorzugsweise eine gegebenenfalls durch Halogen, insbesondere Chlor oder Fluor, und/oder Trifluormethyl mono-, di- oder trisubstituierte Chinolinylgruppe.

Im Rahmen dieses Aspektes werden die nachfolgend aufgeführten Verbindungen besonders bevorzugt:

4-[(Z)-2-[2,7-Bis(trifluormethyl)-4-chinolinyl]vinyl]-4,8-dimethyl-2,3-dioxabicyclo[3.3.1]nonan-7-on,
4-[(Z)-2,4-Dichlorstyryl]-4,8-dimethyl-2,3-dioxabicyclo[3.3.1]nonan-7-on,
4-[(Z)-2,4-Bis(trifluormethyl)styryl]-4,8-dimethyl-2,3-dioxabicyclo[3.3.1]nonan-7-on und
4,8-Dimethyl-4-[(E)-2,3,4,5,6-pentafluorstyryl]-2,3-dioxabicyclo[3.3.1]nonan-7-on.

Die neuen Verbindungen der Formel I können erfindungsgemäss dadurch hergestellt werden, dass man

a) eine Verbindung der allgemeinen Formel

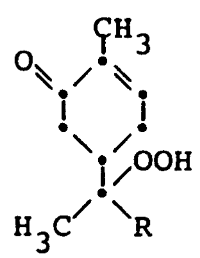

II

worin R obige Bedeutung besitzt,

in Gegenwart einer Base oder einer Säure cyclisiert, oder

b) eine Verbindung der Formel

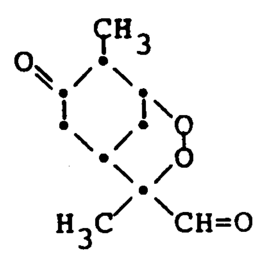

III

mit einem Phosphoran der allgemeinen Formel

$Ø_3P=CH\text{-}R'$ IV

worin Ø Phenyl und R′ Wasserstoff, eine gesättigte oder partiell ungesättigte Kohlenwasserstoffgruppe mit bis zu 13 Kohlenstoffatomen oder eine gegebenenfalls über eine Alkyl- oder Alkenylgruppe mit bis zu 5 Kohlenstoffatomen gebundene Aryl-, Heteroaryl-, Arylcarbonyl-oder Heteroarylcarbonylgruppe bedeuten,
umsetzt, oder
c) die Doppelbindung in einer Verbindung der allgemeinen Formel

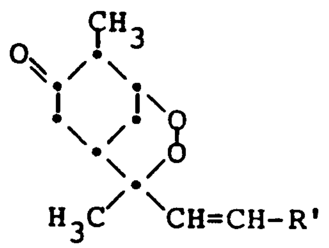

Ia

worin R′ obige Bedeutung besitzt,
reduziert.

Gemäss Verfahrensvariante a) können die Verbindungen der Formel I hergestellt werden, indem man ein Hydroperoxid der allgemeinen Formel II in Gegenwart einer Base oder einer Säure cyclisiert. Diese Cyclisierung wird vorzugsweise in einem organischen Lösungsmittel durchgeführt, wobei z.B. niedere Alkohole, wie Methanol und Aethanol, offenkettige und cyclische Aether, wie Diäthyläther, t-Butylmethyläther und Tetrahydrofuran, halogenierte Kohlenwasserstoffe, wie Methylenchlorid und Chloroform, Dimethylacetamid, Dimethylformamid, Aceton und Acetonitril für diesen Zweck in Frage kommen. Als Lösungsmittel verwendet man vorzugsweise niedere Alkohole, insbesondere Methanol, oder Acetonitril. Geeignete Basen sind die tertiären Amine, wie Triäthylamin, und die niederen Alkalimetallalkoholate, wie Natriummethylat, wobei die letzteren bevorzugt werden. Geeignete Säuren sind Mineralsäuren, wie Salzsäure, Schwefelsäure und Phosphorsäure, organische Sulfonsäuren, beispielsweise niedere Alkylsulfonsäuren, wie Methansulfonsäure, und aromatische Sulfonsäuren, wie Benzolsulfonsäure, Mesitylensulfonsäure und p-Toluolsulfonsäure, und Lewis-Säuren, wie Bortrifluorid. Als Säure verwendet man vorzugsweise die p-Toluolsulfonsäure. Die Cyclisierung wird vorzugsweise in einem Temperaturbereich von etwa 0° bis etwa 40° C durchgeführt.

In einer bevorzugten Ausführungsform verwendet man p-Toluolsulfonsäure in Acetonitril und arbeitet bei Raumtemperatur.

Bei der Cyclisierung erhaltene Gemische von Stereoisomeren, insbesondere von Epimeren in bezug auf die 4-Stellung, können mittels chromatographischer Methoden oder mittels Kristallisation aufgetrennt werden.

Durch Umsetzung eines Aldehydes der Formel III mit einem Phosphoran der Formel IV gemäss Verfahrensvariante b) können Verbindungen der Formel I hergestellt werden, worin R die Gruppe $-CH=CH-R'$ bedeutet, und R′ obige Bedeutung besitzt. Stabile Phosphorane können dabei direkt mit einem Aldehyd der Formel III umgesetzt werden, wobei als Lösungsmittel jedes inerte organische Lösungsmittel in Frage kommt. Vorzugsweise verwendet man offenkettige und cyclische Aether, wie Diäthyläther, t-Butylmethyläther und Tetrahydrofuran, oder halogenierte niedere Kohlenwasserstoffe, wie Methylenchlorid und Chloroform. Die übrigen Phosphorane müssen vorgängig aus den entsprechenden Phosphoniumsalzen und einer starken Base in situ hergestellt werden. Als Base kann man beispielsweise ein niederes Alkyllithium, wie n-Butyllithium, Natriumhydrid oder Natrium-bis-trimethylsilylamid verwenden. Als Lösungsmittel kommen in diesem Fall insbesondere offenkettige oder cyclische Aether, wie Diäthyläther, t-Butylmethyläther und Tetrahydrofuran, in Frage. Die obige Reaktion wird vorzugsweise bei Temperaturen zwischen etwa 0° und etwa 50° C durchgeführt.

Erhaltene Gemische von Isomeren können mittels chromatographischer Methoden oder mittels Kristallisation aufgetrennt werden.

Durch Reduktion, z.B. durch katalytische Hydrierung oder durch Reduktion mit Diimid, einer Verbindung der Formel Ia gemäss Verfahrensvariante c) können Verbindungen der Formel I hergestellt werden, worin R die Gruppe $-CH_2-CH_2-R'$ bedeutet und R′ die obige Bedeutung besitzt. Die katalytische Hydrierung erfolgt nach an sich bekannten und jedem Fachmann geläufigen Methoden. Als Katalysatoren verwendet man vorzugsweise Edelmetallkatalysatoren, insbesondere Platinkatalysatoren, z.B. 5-proz. Platin auf Aktivkohle.

Als Lösungsmittel verwendet man vorzugsweise niedere Fettsäureester, wie Essigester, niedere Alkohole, wie Methanol und Aethanol, und offenkettige oder cyclische Aether, wie Diäthyläther, t-Butylmethyläther und Tetrahydrofuran. Die Hydrierung wird vorzugsweise unter Normaldruck oder wenig darüber durchgeführt. Die Reaktionstemperatur liegt vorzugsweise in einem Bereich von etwa 0° bis etwa 50°C, vorzugsweise bei etwa Raumtemperatur.

Die Reduktion mit Diimid wird in an sich bekannter Weise durchgeführt, indem man das Diimid in situ aus dem Dikaliumsalz der Azodicarbonsäure und einer Säure, beispielsweise einer niederen Fettsäure, wie Essigsäure, herstellt. Man verwendet dabei ein polares Lösungsmittel, vorzugsweise einen niederen Alkohol, wie Methanol und Aethanol. Die Reaktionstemperatur liegt vorzugsweise zwischen etwa -10° und etwa 30°C.

Die als Ausgansstoff verwendeten Verbindungen der Formel II können gemäss den nachfolgenden Reaktionsschemata I und II hergestellt werden; Ra bedeutet dabei eine gesättigte oder partiell ungesättigte Kohlenwasserstoffgruppe mit bis zu 14 Kohlenstoffatomen oder eine gegebenenfalls über eine Alkyl- oder Alkenylgruppe mit bis zu 6 Kohlenstoffatomen gebundene Aryl-, Heteroaryl-, Arylcarbonyl- oder Heteroarylcarbonylgruppe, und R′ besitzt die obige Bedeutung.

Schema I

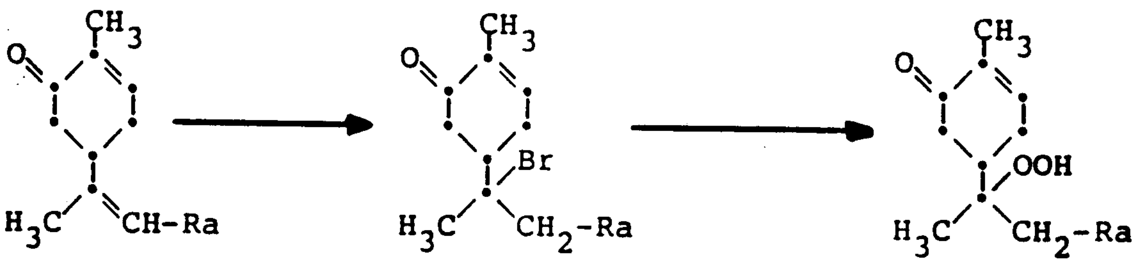

Va VIa IIa

Die Umsetzung einer Verbindung der Formel Va zu einer Verbindung der Formel VIa kann durch Behandeln mit Bromwasserstoff in Gegenwart einer katalytischen Menge einer Lewis-Säure, wie Zinkbromid, in einem organischen Lösungsmittel durchgeführt werden. Als Lösungsmittel eignen sich insbesondere halogenierte niedere Kohlenwasserstoffe, wie Methylenchlorid. Diese Hydrobromierung erfolgt vorzugsweise in einem Temperaturbereich von etwa 0°C bis etwa Raumtemperatur.

Die Umsetzung einer Verbindung der Formel VIa zu einer Verbindung der Formel IIa kann durch Behandeln mit 100-proz. Wasserstoffperoxid in Gegenwart von Silbersalzen, wie Silbertrifluoracetat, in einem inerten organischen Lösungsmittel durchgeführt werden. Als Lösungsmittel eignen sich insbesondere offenkettige und cyclische Aether, wie Diäthyläther, t-Butylmethyläther und Tetrahydofuran. Die Reaktion wird vorzugsweise in einem Bereich von etwa 0°C bis etwa Raumtemperatur durchgeführt.

Schema II

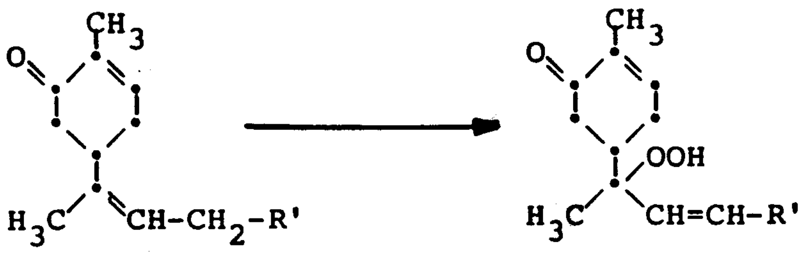

Vb IIb

Die Umsetzung einer Verbindung der Formel Vb zu einer Verbindung der Formel IIb kann in an sich bekannter Weise durch Singlettsauerstoffoxidation bewerkstelligt werden. Diese Reaktion wird vorzugsweise in einem geeigneten organischen Lösungsmittel unter Einleiten von Sauerstoff oder Luft in Gegenwart eines Farbstoffes, wie z.B. Methylenblau, Bengalrosa oder Tetraphenylporphyrin, und unter Bestrahlung mit einer Lampe durchgeführt. Geeignete Lösungsmittel sind z.B. halogenierte niedere Kohlenwasserstoffe, wie Methylenchlorid, niedere Alkohole, wie Methanol und Aethanol, niedere Fettsäureester, wie Essigester, und Acetonitril. Die Reaktion wird vorzugsweise in einem Temperaturbereich von etwa -50 ° C bis etwa Raumtemperatur durchgeführt.

Bei den obigen Verfahren zur Herstellung von Verbindungen der Formel II ist es nicht notwendig, dieselben zu isolieren. Sie können durch Zugabe des Cyclisierungskatalysators, vorzugsweise einer Säure, inbesondere der p-Toluolsulfonsäure, zur erhaltenen Reaktionslösung direkt zu Verbindungen der Formel I umgesetzt werden.

Die als Ausgangsstoffe verwendeten Verbindungen der Formel III können aus Verbindungen der Formel Ia gemäss dem nachfolgenden Reakionsschema III hergestellt werden, worin R′ obige Bedeutung besitzt.

Schema III

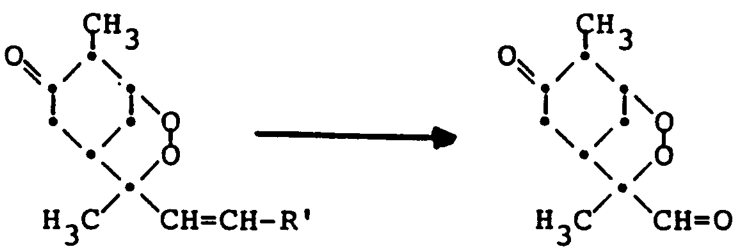

Ia III

Die Umsetzung einer Verbindung der Formel Ia zu einer Verbindung der Formel III kann beispielsweise durch Oxidation mit Ozon bewerkstelligt werden. Es handelt sich dabei um eine an sich bekannte und jedem Fachmann geläufige Umsetzung. Als Lösungsmittel verwendet man vorzugsweise einen niederen Alkohol, wie Methanol, oder einen niederen halogenierten Kohlenwasserstoff, wie Methylenchlorid. Man arbeitet vorzugweise bei tiefen Temperaturen, beispielsweise bei etwa -70 ° C. Als Ausgangsstoff verwendet man vorzugsweise eine Verbindung der Formel Ia, worin R′ Wasserstoff bedeutet.

Die Verbindungen der Formeln II und III sind neu und ebenfalls Gegenstand der vorliegenden Erfindung.

Die übrigen als Ausgangsstoffe verwendeten Verbindungen sind bekannt oder können in Analogie zu den bekannten Vertretern dieser Stoffklassen hergestellt werden. Die fraglichen Verbindungen können insbesondere ausgehend von (5S)-(+)-Carvon oder (5R)-(-)-Carvon nach an sich bekannten Verfahren

hergestellt werden. Die Beispiele, welche weiter unten folgen, enthalten detaillierte Angaben betreffend die Herstellung der fraglichen Ausgangsstoffe.

Wie bereits eingangs erwähnt besitzen die Verbindungen der Formel I wertvolle pharmakologische Eigenschaften. Sie zeigen eine ausgeprägte Wirkung gegen die Erreger der Malaria. Sie weisen eine ausgeprägte blutschizontocide Aktivität auf. Die Wirkung der erfindungsgemässen Substanzen kann beispielsweise im nachfolgend beschriebenen Tierversuch ermittelt werden:

Als Versuchstiere verwendet man männliche, 18-20 g schwere Albinomäuse. Sie werden in klimatisierten Räumen bei 22-23 °C gehalten, und zwar in Gruppen von 5 Tieren pro Käfig. Sie erhalten ad libitum ein Diätfutter mit geringem PABA-Gehalt und Trinkwasser.

Am ersten Versuchstag (DO) werden die Versuchstiere mit Plasmodium berghey infiziert, indem man den Versuchstieren jeweils 0,2 ml heparinisiertes Blut von infizierten Spendermäusen intravenös injiziert. Das Spenderblut wird dabei so verdünnt, dass es pro 0,2 ml $10^7$ parasitierte Erythrozyten enthält. In unbehandelten Kontrolltieren erreicht die Parasitamie am vierten Tag nach der Infektion (D + 4) regelmässig 70-80%, und die Versuchstiere sterben zwischen den Tagen +5 bis +8.

Die zu testenden Substanzen werden in destilliertem Wasser oder in einer Mischung aus Tween 80, Alkohol (96%) und Wasser gelöst oder suspendiert. In der Regel werden je 0,5 ml dieser Lösung oder Suspension subkutan an Gruppen von 5 Versuchstieren verabreicht. Die Behandlung erfolgt erstmals 3 Stunden nach der Infektion, sowie an den drei nachfolgenden Tagen. Zur Titration der Wirkung kommen geeignete Verdünnungen der Testverbindungen zur Anwendung. Pro Versuch werden 10 Tiere in gleicher Weise mit dem Lösungs- oder Suspensionsmedium behandelt.

24 Stunden nach der letzten Behandlung (D + 4) werden von allen Tieren Blutausstriche mit Blut aus Schwanzvenen angefertigt und mit Giemsa gefärbt. Die mittlere Erythrozyten-Infektionsrate (Parasitamie in %) in der Kontrollgruppe, sowie in den Gruppen, welche mit den zu testenden Verbindungen behandelt worden sind, wird durch Auszählung unter dem Mikroskop bestimmt. Die Differenz des Mittelwertes der Infektionsrate zwischen Kontrollgruppe (100%) und den behandelten Gruppen wird errechnet und als Prozent Reduktion ausgedrückt. Aus den erhaltenen Resultaten wird die $ED_{50}$ rechnerisch ermittelt. Die $ED_{50}$ in mg/kg ist diejenige Dosis, welche nach viermaliger Verabreichung die mittlere Erythrozyten-Infektionsrate im Vergleich zur Kontrollgruppe auf 50% reduziert.

In der nachfolgenden Tabelle werden die Resultate zusammengestellt, welche mit repräsentativen Vertretern der durch die Formel I definierten Verbindungsklasse in dem vorstehend geschilderten Versuchs erhalten worden sind. Die Tabelle enthält ausserdem Angaben über die Toxizität einiger dieser Verbindungen. Bei einmaliger oraler Verabreichung der in der Tabelle angegebenen Menge an Mäusen konnten keine toxischen Symptome beobachtet werden.

Tabelle

| Verbindung der Formel I | | Wirkung ED$_{50}$ in mg/kg s.c. | Toxizität Dosis in mg/kg p.o. |
|---|---|---|---|
| R | Konfiguration | | |
| $-(CH_2)_7-CH_3$ | 1R,4S,5S,8R | 5,2 | |
| " | 1R,4R,5S,8R | 6,4 | |
| $-(CH_2)_8-CH_3$ | 1R,4S,5S,8R | 6,0 | |
| " | 1R,4R,5S,8R | 3,6 | |
| $-(CH_2)_9-CH_3$ | 1R,4S,5S,8R | 3,4 | |
| " | 1R,4R,5S,8R | 5,2 | |
| $-(CH_2)_{10}-CH_3$ | 1R,4S,5S,8R | 3,4 | 3000 |
| " | 1R,4R,5S,8R | 5,2 | 3000 |
| " | 1S,4R,5R,8S | 4,6 | |
| " | 1S,4S,5R,8S | 5,9 | |
| $-CH{=}CH-(CH_2)_7-CH_3$ | 1R,4R,5S,8R | 6,2 | |
| " | 1R,4S,5S,8R | 4,5 | |
| 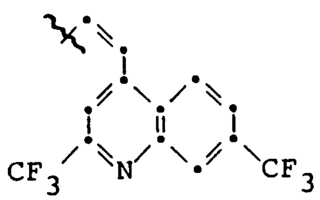 | 1R,4S,5S,8R | 6,2 | 6000 |
| | 1S,4R,5R,8S | 5,4 | |
| 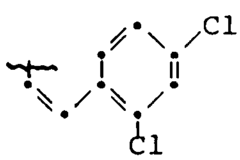 | 1S,4R,5R,8S | 4,6 | |

Die Verbindungen der Formel I können als Heilmittel, z.B. in Form pharmazeutischer Präparate zur enteralen oder parenteralen Applikation, Verwendung finden. Sie können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die beschriebenen Verbindungen der Formel I, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragées und Hartgelatinekapseln beispielsweise Lactose. Maisstärke oder

Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Stabilisierungs-, Konservierungs-, Netz- und Emulgiermittel, Mittel zur geschmacklichen Verbesserung, Salze zur Veränderung des osmotischen Drukkes, Puffersubstanzen, Lösungsvermittler, Färbe- und Ueberzugsmittel und Antioxidantien in Frage.

Die Dosierung der Verbindungen der Formel I kann, abhängig vom zu bekämpfenden Parasiten, dem Alter und dem individuellen Zustand des Patienten und von der Applikationsweise, innerhalb weiter Grenzen variieren und ist natürlich in jedem einzelnen Fall den individuellen Gegebenheiten anzupassen. Zur Verhütung und Behandlung der Malaria kommt für den erwachsenen Patienten eine tägliche Dosis von etwa 0.01 g bis etwa 4 g, insbesondere etwa 0,05 g bis etwa 2 g in Betracht. Je nach Dosierung ist es dabei zweckmässig, die Tagesdosis in mehreren Dosierungseinheiten zu verabreichen.

Die pharmazeutischen Präparate enthalten zweckmässigerweise etwa 10-1000 mg, vorzugsweise 50-500 mg einer Verbindung der Formel 1.

Die nachfolgenden Beispiele dienen der näheren Erläuterung der vorliegenden Erfindung. Sämtliche Temperaturen sind in Celsiusgraden angegeben.

Beispiel 1

a) Eine Lösung von 941 g 3-Chlorperbenzoesäure (55%) in 9 l Methylenchlorid wird im Eisbad abgekühlt. 360 ml (5S)-(+)-Carvon werden so dazugetropft, dass die Temperatur nicht über 20° steigt. Die Reaktionsmischung wird während 3,5 Stunden bei Raumtemperatur gerührt, wobei sich ein Niederschlag von 3-Chlorbenzoesäure bildet. Diese Suspension wird während 30 Minuten gerührt und mit Eis gekühlt, um die Ausfällung zu vervollständigen. Der Niederschlag wird abfiltriert und mit Hexan/Methylenchlorid (9:1) gewaschen. Das Filtrat wird vorsichtig eingedampft. Das so erhaltene Oel (Epoxid) wird in 3 l Eiswasser suspendiert und unter Kühlen im Eisbad so mit 180 ml 3N Schwefelsäure versetzt, dass die Temperatur nicht über 20° steigt. Die Mischung wird während 15 Stunden bei Raumtemperatur gerührt. Der pH wird durch Zugabe von 180 ml 3N Natronlauge auf 6,5 eingestellt. Eine kleine Menge von 3-Chlorbenzoesäure wird abfiltriert. Die wässrige Phase wird auf 0° gekühlt, worauf man 490 g Natrium(meta)perjodat in Portionen über 30 Minuten so zugibt, dass die Temperatur nicht über 20° steigt. Nach 2 Stunden Rühren bei Raumtemperatur werden 30 g Natriumsulfit und 200 g Natriumbicarbonat nacheinander zugegeben. Die Suspension wird filtriert, und das Filtrat wird dreimal mit je 3 l Methylenchlorid extrahiert. Die vereinigten organischen Phasen werden über Natriumsulfat getrocknet und eingedampft. Das Rohprodukt wird aus Hexan/Essigester bei 0° kristallisiert. Man erhält (5S)-5-Acetyl-2-methyl-2-cyclohexen-1-on.Smp. 35°; $[\alpha]_D^{25}$ = 81° (c=1 in Aethanol).

b) 44 g n-Butyltriphenylphosphoniumbromid werden unter Argon in 500 ml trockenem Tetrahydrofuran suspendiert und auf -50° abgekühlt. 69 ml 1,33N n-Butyllithium in Hexan werden dazugegeben. Die orangefarbene Suspension wird auf Raumtemperatur erwärmt und dann wieder auf -50° abgekühlt. 14 g (5S)-5-Acetyl-2-methyl-2-cyclohexen-1-on, gelöst in 20 ml trockenem Tetrahydrofuran, werden über einen Zeitraum von 20 Minuten dazugetropft. Man entfernt dann das Kühlbad und lässt die Suspension auf Raumtemperatur aufwärmen. Nach 2 Stunden Rühren wird die Reaktionsmischung durch Kieselgur filtriert. Das Filtrat wird eingedampft, der dunkle Rückstand wird in 500 ml Essigester gelöst und zweimal mit je 200 ml Wasser gewaschen. Die organische Phase wird über Natriumsulfat getrocknet und eingedampft. Das so erhaltene Oel wird durch Säulenchromatographie an Kieselgel mit Hexan/Essigester (95:5) als Elutionsmittel gereinigt. Man erhält (5S)-2-Methyl-5-[(Z)-1-methyl-1-pentenyl]-2 -cyclohexen-1-on, der noch etwa 15% des entsprechenden (E)-Isomeren enthält.

$^1$H-NMR-Spektrum ($CDCl_3$): u.a. Signale bei 3,1 (m,1H), 5,2 (t,1H) und 6,75 (breit,1H) ppm.

c) 2 g (5S)-2-Methyl-5-[(Z)-1-methyl-1-pentenyl]-2 -cyclohexen-1-on werden in 100 ml Methylenchlorid gelöst und auf 0° abgekühlt. Nach Zugabe einer katalytischen Menge (100 mg) an Zinkchlorid wird die Lösung mit Bromwasserstoffgas gesättigt. Nach 30 Minuten wird die Lösung durch 1 g Kieselgel filtriert und vorsichtig eingedampft. Der erhaltene ölige Rückstand von 2,8 g wird in trockenem Aether gelöst, auf 0° gekühlt und mit 1 ml 100-proz. Wasserstoffperoxid versetzt. 3,45 g Silbertrifluoracetat, gelöst in 25 ml trockenem Aether, werden tropfenweise über eine Zeitraum von 30 Minuten zugegeben. Der

Ueberschuss an Silberionen wird durch Zugabe von 1 ml 1N Salzsäure gefällt, und die erhaltene Suspension wird durch Kieselgel filtriert. Zur Entfernung des Ueberschusses an Wasserstoffperoxid wird das Filtrat fünfmal mit je 10 ml Wasser gewaschen. Die Aetherphase wird dann vorsichtig eingedampft, und der Rückstand wird in 100 ml Methanol aufgenommen. Zu dieser Lösung wird eine katalytische Menge an Natriummethanolat (100 mg) gegeben, worauf man während 15 Stunden bei Raumtemperatur stehen lässt. Das Methanol wird abdestilliert und der Rückstand wird durch Chromatographie an Keiselgel unter Eluieren mit Hexan/Essigester (7:3) gereinigt und in die beiden Diastereomeren (1:1) aufgetrennt. Man erhält (1R,4R,5S,8R)-4-Butyl-4,8-dimethyl -2,3-dioxabicyclo[3.3.1]nonan-7-on [Massenspektrum: u.a. Spitzen bei m/e 227 ($M^+$ +1), 153 und 85; $[\alpha]_D^{25}$ = -137,5° (c=1 in Methanol)] und (1R,4S,5S,8R)-4-Butyl-4,8-dimethyl -2,3-dioxabicyclo[3.3.1)-nonan-7-on [Masspektrum: u.a. Spitzen bei m/e 227 ($M^+$ +1), 153 und 85].

In analoger Weise erhält man:

d) Unter Verwendung von (5R)-(-)-Carvon anstelle von (5S)-(+)-Carvon als Ausgansstoff das (1S,4R,5R,8S)-4-Butyl-4,8-dimethyl-2,3 -dioxabicyclo[3.3.1]nonan-7-on [Massenspektrum: u.a. Spitzen bei m/e 227 ($M^+$ +1), 153 und 85] und das (1S,4S,5R,8S)-4-Butyl-4,8-dimethyl-2,3 -dioxabicyclo[3.3.1]nonan-7-on [Massenspektrum: u.a. Spitzen bei m/e 227 ($M^+$ +1), 153 und 85];

e) unter Verwendung von Isopentyltriphenylphosphoniumbromid in Stufe b) anstelle von n-Butyltriphenylphosphoniumbromid das (1R,4S,5S,8R)-4-Isopentyl-4,8-dimethyl-2,3 -dioxabicyclo]3.3.1]nonan-7-on [$^1$H-NMR-Spektrum ($CDCl_3$): u.a. Signale bie 0,9 (d,6H), 1,05 (d,3H) und 1,1 (s,3H) ppm] und das (1R,4R,5S,8R)-4-Isopentyl-4,8-dimethyl-2,3 -dioxabicyclo[3.3.1]nonan-7-on [$^1$H-NMR-Spektrum ($CDCl_3$): u.a. Signale bei 0,8 (d,6H), 1,3 (d,3H) und 1,4 (s,3H) ppm];

f) unter Verwendung von 3-Phenylpropyltriphenylphosphoniumbromid in Stufe b) anstelle von n-Butyltriphenylphosphoniumbromid das (1R,4S,5S,8R)-4,8-Dimethyl-4-(3-phenylpropyl)-2,3 -dioxabicyclo[3.3.1]-nonan-7-on [Massenspektrum (chemische Ionisation): u.a. Spitzen bei m/e 256 ($M^+$-32) und 104] und das (1R,4R,5S,8R)-4,8-Dimethyl-4-(3-phenylpropyl)-2,3 -dioxabicyclo[3.3.1]nonan-7-on [Massenspektrum (chemische Ionisation): u.a. Spitzen bei m/e 306 ($M^+$ +$NH_4$), 273 ($M^+$-15) und 180];

g) unter Verwendung von (5R)-(-)-Carvon als Ausgansstoff anstelle von (5S)-(+)-Carvon und von 3-Phenylpropyltriphenylphosphoniumbromid in Stufe b) anstelle von n-Butyltriphenylphosphoniumbromid das (1S,4R,5R,8S)-4,8-Dimethyl-4-(3-phenylpropyl)-2,3 -dioxabicyclo[3.3.1]nonan-7-on [$^1$H-NMR-Spektrum ($CDCl_3$): u.a. Signale bei 0,9 (s,3H) und 7,2 (s,5H) ppm] und das (1S,4S,5R,8S)-4,8-Dimethyl-4-(3-phenylpropyl)-2,3 -dioxabicyclo[3.3.1]nonan-7-on [$^1$H-NMR-Spektrum ($CDCl_3$): u.a. Signale bie 1,3 (s,3H) und 7,2 (s,5H) ppm];

h) durch direkt Umsetzung von (5S)-(+)-Carvon gemäss Stufe c) das (1R,5S,8R)-4,4,8-Trimethyl-2,3-dioxabicyclo[3.3.1]nonan-7-on [Massenspektrum: u.a. Spitzen bei m/e 184 ($M^+$), 126 und 85];

i) durch direkte Umsetzung von (5R)-(-)-Carvon gemäss Stufe c) das (1S,5R,8S)-4,4,8-Trimethyl-2,3-dioxabicyclo[3.3.1]nonan-7-on [Massenspektrum: u.a. Spitzen bei m/e 184 ($M^+$) und 85].

## Beispiel 2

a) 4,5 g Aethyltriphenylphosphoniumbromid und 1,8 ml N,N,N′,N′-Tetramethyl-äthylendiamin werden in 300 ml Tetrahydrofuran unter Argon auf 0° abgekühlt. Dann tropft man unter Rühren 7,5 ml n-Butyllithium (1,6M in Hexan) dazu und rührt noch 1 Stunde ohne Eiskühlung. Die rote Lösung wird auf -70° abgekühlt, worauf 1,52 g (S)-5-Acetyl-2-methyl-2-cyclohexen-1-on, gelöst in 10 ml Tetrahydrofuran, während 15 Minuten dazugetropft werden. Man entfernt das Kühlbad und rührt noch 45 Minuten weiter. Nach Zugabe von 20 ml Wasser wird eingedampft. Der Rückstand wird in Aether aufgenommen und je zweimal mit 0,3N Schwefelsäure und gesättigter Kochsalzlösung extrahiert. Die Aetherphase wird über Natriumsulfat getrocknet und eingedampft. Das Rohprodukt wird durch Blitzchromatographie an Kieselgel unter Eluieren mit Hexan/Methylenchlorid (1:1) gereinigt. Man erhält (5S)-2-Methyl-5-[(Z)-1-methylpropenyl]-2-cyclohexen-1-on als farbloses Oel [Massenspektrum: u.a. Spitzen bei m/e 164 ($M^+$), 149 ($M^+$-15,$CH_3$), 135 ($M^+$-29,COH), 82 und 54].

48 g (5S)-2-Methyl-5-[(Z)-1-methylpropenyl]-2 -cyclohexen-1-on und 150 mg Methylenblau, gelöst in 400 ml Acetonitril, werden in einem mit einer 600W-Weisslichtlampe, einem Umwälzrührwerk und einer Kryostatköhlung (-30°) versehenen Photooxidator unter leichtem $O_2$-Strom während 22 Stunden bei 0° belichtet. Das Reaktionsgemisch wird aus der Apparatur entfernt, mit 1 g Toluol-4-sulfonsäure-monohydrat versetzt und während 2 Stunden bei Raumtemperatur gerührt. Nach Zugabe von 20 ml Dimethylsulfid wird das Reaktionsgemisch vorsichtig eingedampft, und der Rückstand wird durch Säulenchromatographie an Keiselgel mit Methylenchlorid als Elutionsmittel gereinigt. Man erhält (1R,4RS,5S,8R)-4,8-Dimethyl-4-vinyl-2,3 -dioxabicyclo[3.3.1]nonan-7-on als farbloses Oel [Masenspektrum (chemische Ionisation): u.a. Spitzen bei m/e 214 ($M^+ + NH_4$) und 197 ($M^+ + 1$)].

Das nach dem Belichten erhaltene Reaktionsgemisch enthält (5S)-5-[(RS)-2-Hydroperoxy-3-buten-2-yl]-2-methyl -2-cyclohexen-1-on, das durch Eindampfen und Blitz-Chromatographie des Rückstandes an Keiselgel unter Eluieren mit Hexan/Essigester (3:1) isoliert und gereinigt werden kann [Massenspektrum (chemische Ionisation): u.a. Spitzen bei m/e 214 ($M^+ + NH_4$), 198, 179 und 163].

Ausgehend von entsprechenden Ausgansstoffen, welche gemäss den Stufen a) und b) von Beispiel 1 mit den jeweils angegebenen Aenderungen erhalten werden, erhält man in analoger Weise:

b) das (1R,4RS,5S,8R)-4-[(E)-1-butenyl]-4,8-dimethyl-2,3 -dioxabicyclo[3.3.1]nonan-7-on [$^1$H-NMR-Spektrum ($CDCl_3$): u.a. Signale bei 5,55 (m,2H) und 4,25 (breit,1H) ppm; Massenspektrum (chemische Ionisation): u.a. Spitzen bei m/e 242 ($M^+ + NH_4$), 144 und 116].

c) unter Verwendung von Octyltriphenylphosphoniumbromid in Stufe b) von Beispiel 1 anstelle von n-Butyltriphenylphosphoniumbromid das (1R,4R,5S,8R)-4,8-Dimethyl-4-[(E)-1-octenyl]-2,3 - dioxabicyclo-[3.3.1]nonan-7-on [Massenspektrum: u.a. Spitzen bei m/e 155 und 71] und das (1R,4S,5S,8R)-4,8-Dimethyl-4-[(E)-1-octenyl]-2,3 -dioxabicyclo[3.3.1]nonan-7-on [Massenspektrum: u.a. Spitzen bei m/e 249, 139, 55 und 43];

d) unter Verwendung von Nonyltriphenlphosphoniumbromid in Stufe b) von Beispiel 1 anstelle von n-Butyltriphenylphosphoniumbromid das (1R,4R,5S,8R)-4,8-Dimethyl-4-[(E)-1-nonenyl]-2,3 -dioxabicyclo-[3.3.1]nonan-7-on [$^1$H-NMR-Spektrum ($CDCl_3$): u.a. Signale bei 1,10 (s,3H), 4,35 (m,1H) und 5,2-6,0 (m,2H) ppm] und das (1R,4S,5S,8R)-4,8-dimethyl-4-[(E)-1-nonenyl]-2,3 -dioxabicyclo[3.3.1]nonan-7-on [Massenspektrum: u.a. Spitzen bei m/e 169 und 71];

e) unter Verwendung von Decycltriphenylphosphoniumbromid in Stufe b) von Beispiel 1 anstelle von n-Butyltriphenylphosphoniumbromid das (1R,4R,5S,8R)-4-[(E)-1-Decenyl]-4,8-dimethyl-2,3 -dioxabicyclo-[3.3.1]nonan-7-on [Massenspektrum: u.a. Spitzen bei m/e 309 ($M^+ + 1$) und 71] und das (1R,4S,5S,8R)-4-[(E)-1-Decenyl]-4,8-dimethyl-2,3 -dioxabicyclo[3.3.1]nonan-7-on [Massenspektrum: u.a. Spitzen bei m/e 183 und 71];

f) unter Verwendung von Undecyltriphenylphosphoniumbromid in Stufe b) von Beispiel 1 anstelle von n-Butyltriphenylphosponiumbromid das (1R,4R,5S,8R)-4,8-Dimethyl-4-[(E)-1-undecenyl]-2,3 -dioxabicyclo-[3.3.1]nonan-7-on [Massenspektrum: u.a. Spitzen bei m/e 236, 197, 181 und 71] und das (1R,4S,5S,8R)-4,8-Dimethyl-4-[(E)-1-undecenyl]-2,3 -dioxabicyclo[3.3.1]nonan-7-on [Massenspektrum: u.a. Spitzen bei m/e 304 ($M^+$-18, $H_2O$), 197, 55 und 43];

g) unter Verwendung von (5R)-(-)-Carvon als Ausgansstoff in Stufe a) von Beispiel 1 anstelle von (5S)-(+)-Carvon und von Undecyltriphenylphosphoniumbromid in Stufe b) von Beispiel 1 anstelle von n-Butyltriphenylphosphoniumbromid das (1S,4S,5R,8S)-4,8-Dimethyl-4-[(E)-1-undecenyl]-2,3 -dioxabicyclo-[3.3.1]nonan-7-on [Massenspektrum: u.a. Spitzen bei m/e 236, 197, 181 und 71] und das (1S,4R,5R,8S)-4,8-Dimethyl-4-[(E)-1-undecenyl]-2,3 -dioxabicyclo[3.3.1]nonan-7-on [Massenspektrum: u.a. Spitzen bei m/e 304 ($M^+$-18, $H_2O$), 197, 55 und 43];

h) unter Verwendung von Dodecyltriphenylphosponiumbromid in Stufe b) von Beispiel 1 anstelle von n-Butyltriphenylphosphoniumbromid das (1R,4R,5S,8R)-4-[(E)-1-Dodecenyl]-4,8-dimethyl-2,3 -dioxabicyclo-[3.3.1]nonan-7-on [Massenspektrum: u.a. Spitzen bei m/e 211 und 71] und das (1R,4S,5S,8R)-4-[(E)-1-Dodecenyl]-4,8-dimethyl-2,3 -dioxabicyclo[3.3.1]nonan-7-on [Massenspektrum: u.a. Spitzen bei m/e 318 ($M^+$-18), 211 und 71];

i) unter Verwendung von Tetradecyltriphenylphosphoniumbromid in Stufe b) von Beispiel 1 anstelle von n-Butyltriphenylphosphoniumbromid das (1R,4R,5S,8R)-4,8-Dimethyl-4-[(E)-1-tetradecenyl]-2,3 - dioxabicyclo[3.3.1]nonan-7-on [Massenspektrum: u.a. Spitzen bei m/e 288, 239 und 71] und das (1R,4S,5S,8R)-4,8-Dimethyl-4-[(E)-1-tetradecenyl]-2,3 -dioxabicyclo[3.3.1]nonan-7-on [Massenspektrum: u.a. Spitzen bei m/e 346 ($M^+$-18,$H_2O$), 332 ($M^+$-32,$O_2$), 239 und 71].

Beispiel 3

a) 3,59 g (1R,4S,5S,8R)-4-[(E)-1-Decenyl]-4,8-dimethyl -2,3-dioxabicyclo[3.3.1]nonan-7-on [erhalten gemäss Beispiel 2e)], gelöst in 180 ml Essigester, werden über 360 mg 5-proz. Platin/Kohle bei $1{,}013 \cdot 10^5$ Pa mit Wasserstoff während etwa 1,25 Stunden hydriert. Der Endpunkt der Reaktion wird mittels gaschromatographischer Analyse ermittelt. Der Katalysator wird dann mittels Filtration durch Kieselgur entfernt, und das Filtrat wird eingedampft. Der Rückstand wird durch Säulenchromatographie an Kieselgel unter Eluieren mit Hexan/Essigester (3:1) gereinigt. Man erhält (1R,4S,5S,8R)-4-Decyl-4,8-dimethyl-2,3 -dioxabicyclo[3.3.1]nonan-7-on als farbloses Oel, das aus Hexan kristallisiert [Smp. 57°].

In analoger Weise erhält man:

b) aus dem ersten Produkt von Beispiel 2c) das (1R,4S,5S,8R)-4,8-Dimethyl-4-octyl-2,3 -dioxabicyclo-[3.3.1]nonan-7-on [Samp. 41°];

c) aus dem zweiten Produkt von Beispiel 2c) das (1R,4R,5S,8R)-4,8-Dimethyl-4-octyl-2,3 -dioxabicyclo-[3.3.1]nonan-7-on [Smp. 27°];

d) aus dem ersten Produkt von Beispiel 2d) das (1R,4S,5S,8R)-4,8-Dimethyl-4-nonyl-2,3 -dioxabicyclo-[3.3.1]nonan-7-on [Smp. 39°];

e) aus dem zweiten Produkt von Beispiel 2d) das (1R,4R,5S,8R)-4,8-Dimethyl-4-nonyl-2,3 -dioxabicyclo-[3.3.1]nonan-7-on [Smp. 40°];

f) aus dem ersten Produkt von Beispiel 2e) das (1R,4R,5S,8R)-4-Decyl-4,8-dimethyl-2,3 -dioxabicyclo-[3.3.1]nonan-7-on [Smp. 40°];

g) aus dem zweiten Produkt von Beispiel 2f) das (1R,4S,5S,8R)-4,8-Dimethyl-4-undecyl-2,3 -dioxabicyclo[3.3.1]nonan-7-on [Smp. 56°; $[\alpha]_D^{25}$ = -158,8 (c = 1 in Aethanol)];

h) aus dem ersten Produkt von Beispiel 2f) das (1R,4R,5S,8R)-4,8-Dimethyl-4-undecyl-2,3 -dioxabicyclo-[3.3.1]nonan-7-on [Smp. 42°; $[\alpha]_D^{25}$ = -157,7 (c = 1 in Aethanol)];

i) aus dem zweiten Produkt von Beispiel 2g) das (1S,4R,5R,8S)-4,8-Dimethyl-4-undecyl-2,3 -dioxacyclo-[3.3.1]nonan-7-on [Smp. 56°];

j) aus dem ersten Produkt von Beispiel 2g) das (1S,4S,5R,8S)-4,8-Dimethyl-4-undecyl-2,3 -dioxabicyclo-[3.3.1]nonan-7-on [Smp. 42°];

k) aus dem zweiten Produkt von Beispiel 2h) das (1R,4S,5S,8R)-4-Dodecyl-4,8-dimethyl-2,3 -dioxabicyclo[3.3.1]nonan-7-on [Smp. 67°];

l) aus dem ersten Produkt von Beispiel 2h) das (1R,4R,5S,8R)-4-Dodecyl-4,8-dimethyl-2,3 -dioxabicyclo-[3.3.1]nonan-7-on [Smp. 35°];

m) aus dem zweiten Produkt von Beispiel 2i) das (1R,4S,5S,8R)-4,8-Dimethyl-4-tetradecyl-2,3 -dioxabicyclo[3.3.1]nonan-7-on [Smp. 74°];

n) aus dem ersten Produkt von Beispiel 2i) das (1R,4R,5S,8R)-4,8-Dimethyl-4-tetradecyl-2,3 -dioxabicyclo[3.3.1]nonan-7-on [Smp. 46°].

## Beispiel 4

a) 33 g (1R,4RS,5S,8R)-4,8-Dimethyl-4-vinyl-2,3 -dioxabicyclo[3.3.1]nonan-7-on [erhalten gemäss Beispiel 2a)] werden in 100 ml Methanol gelöst und unter Argon auf -70° abgekühlt. Ein Ozonstrom aus 40 l Sauerstoff/h wird eingeleitet und bis zur Sättigung (hellblaue Farbe) aufrechterhalten. Nach 3-4 Stunden wird die Stromquelle abgeschaltet, und der Ueberschuss an Ozon wird durch Sauerstoff verdrängt. 40 ml Dimethylsulfid werden dazugegeben, und dann lässt man die Suspension auf Raumtemperatur aufwärmen. Das Methanol wird abdestilliert, und der Rückstand wird durch Säulenchromatographie an Kieselgel unter Eluieren mit Hexan/Essigester (2:1 bis 2:2) gereinigt und aufgetrennt. Man erhält nach Kristallisation aus Hexan/Essigester bei 0° (1R,4R,5S,8R)-4,8-Dimethyl-7-oxo -2,3-dioxabicyclo[3.3.1.]nonan-4-carboxaldehyd [Smp. 92°] und (1R,4S,5S,8R)-4,8-Dimethyl-7-oxo -2,3-dioxabicyclo[3.3.1.]nonan-4-carboxaldehyd [Smp. 97°].

b) 760 mg (1R,4S,5S,8R)-4,8-Dimethyl-7-oxo -2,3-dioxabicyclo[3.3.1]nonan-4-carboxaldehyd werden in 80 ml Methylenchlorid gelöst. 2,5 g 2,7-Bis(trifluormethyl)chinolin-4-yl -methylidentriphenylphosphoran werden dazugegeben, und die Lösung wird während 24 Stunden bei Raumtemperatur stehen gelassen. Das Methylenchlorid wird abdestilliert, und der Rückstand wird durch Säulenchromatographie an Keiselgel unter Eluieren mit Methylenchlorid in die (E)- und (Z)-Isomeren aufgetrennt. Nach Kristallisation aus heissem Methanol erhält man (1R,4S,5S,8R)-4-[(Z)-2-[2,7-Bis(trifluormethyl) -4-chinolinyl]vinyl]-4,8-dimethyl -2,3-dioxabicyclo[3.3.1]nonan-7-on als weisse Kristalle [Smp. 160°; $[\alpha]_D^{25}$ = 53,7° (c=1 in Aethanol)] und (1R,4S,5S,8R)-4-[(E)]-2-[2,7-Bis(trifluormethyl) -4-chinolinyl]vinyl]-4,8-dimethyl -2,3-dioxabicyclo[3.3.1]nonan-7-on [Smp. 188°].

In analoger Weise erhält man:

c) unter Verwendung eines entsprechenden aus (5R)-(-)-Carvon hergestellten Ausgansstoffes das (1S,4R,5R,8S)-4-[(Z)-2-[2,7-Bis(trifluormethyl) -4-chinolinyl]vinyl]-4,8-dimethyl -1,3-dioxabicyclo[3.3.1]-nonan-7-on [Smp. 160°];

d) unter Verwendung des mittels p-Trifluormethylbenyltriphenylphosponiumbromid und Natrium-bis-trimethylsilyl)amid in situ hergestellten p-Trifluormethylbenzylidentriphenylphosphoranes das (1R,4R,5S,8R)-4,8-Dimethyl-4-[(E)-4-(trifluormethyl)styryl] -2,3-dioxabicyclo[3.3.1]nonan-7-on [Massenspektrum: u.a. Spitzen bei m/e 321 ($M^+$-19,F) und 85];

e) unter Verwendung des mittels p-Chlorphenacyltriphenylphosponiumbromid und Natriumäthylat in situ hergestellten p-Chlorphenacylidentriphenylphosphoranes das (1R,4S,5S,8R)-4-[(E)-2-(4-Chlorbenzoyl}vinyl]-4,8-dimethyl -2,3-dioxabicyclo[3.3.1]nonan-7-on [Smp. 127° ];

f) unter Verwendung des mittels 2-(1-Adamantyl)äthyltriphenylphosphoniumbromidund n-Butyllithium in situ hergestellten 2-(1-Adamantyl)äthylidentriphenylphosphoranes das (1R,4R,5S,8R)-4-[(Z)-3-(1-Adamantyl)propenyl]-4,8-dimethyl -2,3-dioxabicyclo[3.3.1]nonan-7-on [Smp. 146° (Zersetzung)];

g) unter Verwendung des mittels Geranyltriphenylphosphoniumbromid und n-Butyllithium in situ hergestellten Geranylidentriphenylphosphoranes das (1R,4R,5S,8R)-4-[(all-Z)-4,8-Dimethyl-1,3,7-nonatrienyl] -4,8-dimethyl-2,3-dioxabicyclo[3.2.1]nonan-7-on [Massenspektrum: u.a. Spitzen bei m/e 318 ($M^+$) und 69] und das

h) (1R,4S,5S,8R)-4-[(1Z,3E)-4,8-Dimethyl-1,3,7-nonatrienyl] -4,8-dimethyl-2,3-dioxabicyclo[3.2.1]nonan-7-on [Massenspektrum: u.a. Spitzen bei m/e 249 und 69];

i) unter Verwendung eines entsprechenden aus (5R)-(-)-Carvon hergestellten Ausgansstoffes und von 2,8-Bis(trifluormethyl)chinolin-4-yl-methylidentriphenylphosphoran das (1S,4R,5R,8S)-4-(Z)-2-[2,8-Bis-(trifluormethl)-4-chinolinyl]vinyl]-4,8-dimethyl2,3 -dioxabicyclo[3.3.1]nonan-7-on [Smp. 137° ];

k) unter Verwendung eines entsprechenden aus (5R)-(-)-Carvon hergestellten Ausgansstoffes und 6,8-Dichlor-2-trifluormethyl-4-chinolinyl -methylidentriphenylphosphoran das (1S,4R,5R,8S)-4-[(E)-2-[6,8-Dichlor-2-(trifluormethyl)-4 -chinolinyl]vinyl]-4,8-dimethyl-2,3-dioxabicyclo[3.3.1]nonan -7-on [Smp. 196° ] und das

l) (1S,4R,5R,8S)-4-[(Z)-2-[6,8-Dichlor-2-(trifluormethyl)-4 -chinolinyl]vinyl]-4,8-dimethyl-2,3-dioxabicyclo-[3.3.1]nonan -7-on [Smp. 191° ];

m) unter Verwendung des mittels 2,4-Dichlorbenzyltriphenylphosphoniumchlorid und n-Butyllithium hergestellten 2,4-Dichlorbenzylidentriphenylphosphoranes das (1R,4R,5S,8R)-4-[(Z)-2,4-Dichlorstyryl]-4,8-dimethyl-2,3 -dioxabicyclo[3.3.1]nonan-7-on [Massenspektrum (chemische Ionisation): u.a. Spitzen bei m/e 358 ($M^+ + NH_4$) und 179];

n) unter Verwendung des mittels 3,4-Dichlorbenzyltriphenylphosphoniumchlorid und Natrium-bis-(trimethylsilyl)amid hergestellten 3,4-Dichlorbenzylidentriphenylphosphoranes das (1R,4S,5S,8R)-4-[(Z)-3,4-Dichlorstyryl]-4,8-dimethyl-2,3 -dioxabicyclo[3.3.1]nonan-7-on [Smp. 135° ];

o) unter Verwendung eines entsprechenden aus (5R)-(-)-Carvon hergestellten Ausgansstoffes und des mittels 2,4-Dichlorbenzyltriphenylphosphoniumbromid und Natrium-bis(trimethylsilyl)amid hergestellten 2,4-Dichlorbenzylidentriphenylphosphoranes das (1S,4R,5R,8S)-4-[(Z)-2,4-Dichlorstyryl]-4,8-dimethyl-2,3 -dioxabicyclo[3.3.1]nonan-7-on [Smp. 137° ];

p) unter Verwendung eines entsprechenden aus (5R)-(-)-Carvon hergestellten Ausgansstoffes und des mittels 1-Naphthylmethylentriphenylphosphoniumbromid und Natrium-bis(trimethylsilyl)amid hergestellten 1-Naphthylmethylidentriphenylphosphoranes das (1S,4R,5R,8S)-4,8-Dimethyl-4-[(Z)-2-(1-naphthyl)vinyl]-2,3 -dioxabicyclo[3.3.1]nonan-7-on [Massenspektrum: u.a. Spitzen bei m/e 322 ($M^+$)und 152];

q) unter Verwendung eines entsprechenden aus (5R)-(-)-Carvon hergestellten Ausgansstoffes und des mittels 4-Brom-1-naphthylmethylentriphenylphosphoniumbromid und Natrium-bis(trimethylsilyl)amid hergestellten 4-Brom-1-naphthylmethylidentriphenylphosphoranes das (1S,4R,5R,8S)-4-[(Z)-2-(4-Brom-1-naphthyl)vinyl]-4,8-dimethyl -2,3-dioxabicyclo[3.3.1]nonan-7-on [Smp. 167° ];

r) unter Verwendung eines entsprechenden aus (5R)-(-)-Carvon hergestellten Ausgangsstoffes und des mittels Benzyltriphenylphosphoniumbromid und Natrium-bis(trimethylsilyl)amid hergestellten Benzylidentriphenylphosphoranes das (1S,4R,5R,8S)-4,8-Dimethyl-4-[(Z)-styryl]-2,3 -dioxabicyclo[3.3.1]nonan-7-on [Smp. 60° ];

s) unter Verwendung eines entsprechenden aus (5R)-(-)-Carvon hergestellten Ausgansstoffes und des mittels 4-Fluorbenzyltriphenylphosphoniumchlorid und Natrium-bis(trimethylsilyl)amid hergestellten 4-Fluorbenzylidentriphenylphosphoranes das (1S,4R,5R,8S)-4-[(Z)-p-Fluorstyryl]-4,8-dimethyl-2,3 -dioxabicyclo[3.3.1]nonan-7-on [Smp. 84° ];

t) unter Verwendung eines entsprechenden aus (5R)-(-)-Carvon hergestellten Ausgansstoffes und des mittels 4-Chlorbenzyltriphenylphosphoniumchlorid und Natrium-bis(trimethylsilyl)amid hergestellten 4-Chlorbenzylidentriphenylphosphoranes das (1S,4R,5R,8S)-4-[(Z)-4-Chlorstyryl]-4,8-dimethyl -2,3-dioxabicyclo[3.3.2]nonan-7-on [Smp. 90° ];

u) unter Verwendung eines entsprechenden aus (5R)-(-)-Carvon hergestellten Ausgansstoffes und des mittels 4-Methylbenzyltriphenylphosphoniumbromid und Natrium-bis(trimethylsily)amid hergestellten 4-Methylbenzylidentriphenylphosphoranes das (1S,4R,5R,8S)-4,8-Dimethyl-4-[(Z)-4-methylstyryl]-2,3 -dioxabicyclo[3.3.2]nonan-7-on [Massenspektrum: u.a. Spitzen bei m/e 286 ($M^+$) und 145];

v) unter Verwendung eines eintsprechenden aus (5R)-(-)-Carvon hergestellten Ausgansstoffes und des mittels 4-(Trifluormethyl)benzyltriphenylphosphoniumbromid und Natrium-bis(trimethylsilyl)amid hergestellten 4-Trifluormethyl)benzylidentriphenylphosphoranes das (1S,4R,5R,8S)-4,8-Dimethyl-4-[(Z)-4-(trifluormethyl)styryl]-2,3-dioxabicyclo[3.3.1]nonan-7-on [Massenspektrum (chemische Ionisation): u.a.

Spitzen bei m/e 358 ($M^+ + NH_4$) und 340];

w) unter Verwendung eines entsprechenden aus (5R)-(-)-Carvon hergestellten Ausgansstoffes und des mittels 3-(Trifluormethyl)benzyltriphenylphosphoniumchlorid und Natrium-bis(trimethylsilyl)amid hergestellten 3-(Trifluormethyl)benzylidentriphenylphosphoranes das (1S,4R,5R,8S)-4,8-Dimethyl-4-[(Z)-3-(trifluormethyl}styryl]-2,3-dioxa bicyclo[3.3.1]nonan-7-on [Smp. 101 ° ];

x) unter Verwendung eines entsprechenden aus (5R)-(-)-Carvon hergestellten Ausgangsstoffes und des mittels 4-Cyanobenzyltriphenylphosphoniumbromid und Natrium-bis(trimethylsilyl)amid hergestellten 4-Cyanobenzylidentriphenylphosphoranes das 4-[(Z)-2-[(1S,4R,5R,8S)-4,8-Dimethyl-7-oxo-2,3 - dioxabicyclo[3.3.1]nonan-4-yl]vinyl]benzonitril [Smp. 125 ° ];

y) unter Verwendung eines entsprechenden aus (5R)-(-)-Carvon hergestellten Ausgansstoffes und des mittels 4-(t-Butyl)benzltriphenylphosphoniumbromid und Natrium-bis(trimethylsilyl)amid hergestellten 4-(t-Butyl)benzylidentriphenylphosphoranes das (1S,4R,5R,8S)-4-[(Z)-4-(t-Butyl)styryl]-4,8-dimethyl-2,3-dioxabicyclo[3.3.1]nonan-7-on [Smp. 101 ° ];

z) unter Verwendung eines entsprechenden aus (5R)-(-)-Carvon hergestellten Ausgansstoffes und des mittels 4-Phenylbenzyltriphenylphosphoniumchlorid und Natrium-bis(trimethylsilyl)amidhergestellten 4-Phenylbenzylidentriphenylphosphoranes das (1S,4R,5R,8S)-4-[(Z)-4-Biphenylvinyl]4,8-dimethyl -2,3-dioxabicyclo[3.3.1]nonan-7-on [Smp. 148 ° ];

aa) unter Verwendung eines entsprechenden aus (5R)-(-)-Carbon heregestellten Ausgansstoffes und des mittels 4-Methoxybenzyltriphenylphosphoniumchlorid und Natrium-bis(trimethylsilyl)amid hergestellten 4-Methoxybenzylidentriphenylphosphoranes das (1S,4R,5R,8S)-4-[(E/Z)-4-Methoxystyryl)-4,8-dimethyl-2,3 - dioxabicyclo[3.3.1]nonan-7-on, E/Z-Verhältnis = 1:3 [Massenspektrum: u.a. Spitzen bei m/e 302 ($M^+$) und 161];

bb) unter Verwendung eines entsprechenden aus (5R)-(-)-Carvon hergestellten Ausgansstoffes und des mittels 2,4-Dichlorbenzyltriphenylphosponiumchlorid und Natrium-bis(trimethylsily)amid hergestellten 2,4-Dichlorbenzylidentriphenylphosphoranes das (1S,4R,5R,8S)-4-[(Z)-2,4-Dichlorstyryl]-4,8-dimethyl-2,3-dioxabicyclo[3.3.1]nonan-7-on [Massenspektrum (chemische Ionisation): u.a. Spitzen bei m/e 358 ($M^+ + NH_4$) und 179];

cc) unter Verwendung eines entsprechenden aus (5R)-(-)-Carvon hergestellten Ausgangsstoffes und des mittels 2,4-Bis(trifluormethyl)benzyltriphenylphosphoniumbromid und Natrium-bis(trimethylsilyl)amid hergestellten 2,4-Bis(trifluormethyl)benzylidentriphenylphosphoranes das (1S,4R,5R,8S)-4-[(Z)-2,4-Bis-(trifluormethyl)styryl]4,8-dimethyl -2,3-dioxabicyclo[3.3.1]nonan-7-on [Smp. 124 ° ];

dd) unter Verwendung eines entsprechenden aus (5R)-(-)-Carvon hergestellten Ausgansstoffes und des mittels 2-Chlor-4-(trifluormethyl)benzyltriphenylphosphoniumchlorid und Natrium-bis(trimethylsilyl)amid hergestellten 2-Chlor-4-(trifluormethyl)benzylidentriphenylphosphoranes das (1S,4R,5R,8S)-4-[(Z)-2-Chlor-4-(trifluormethyl)styryl]-4,8-dimethyl-2,3 -dioxabicyclo[3.3.1]nonan-7-on [Samp. 160 ° ];

ee) unter Verwendung eines entsprechenden aus (5R)-(-)-Carvon hergestellten Ausgansstoffes und des mittels 3,5-Bis(trifluormethyl)benzyltriphenylphosphoniumbromid und Natrium-bis(trimethylsilyl)amid hergestellten 3,5-Bis(trifluormethyl)benzylidentriphenylphosphoranes das (1S,4R,5R,8S)-4-[(E/Z)-3,5-Bis-(trifluormethyl)styryl]-4,8-dimethyl-2,3 -dioxabicyclo[3.3.1]nonon-7-on, E/Z-Verhältnis = 1.5 [Massenspektrum: u.a. Spitzen bei m/e 389 ($M^+$-F) und 267];

ff) unter Verwendung eines entsprechenden aus (5R)-(-)-Carvon hergestellten Ausgansstoffes und des mittels 2-Chlor-4-cyanobenzyltriphenylphosphoniumbromid und Natrium-bis(trimethylsilyl)amid hergestellten 2-Chlor-4-cyanobenzylidentriphenylphosphoranes das 3-Chlor-4-[(Z)-2-(1S,4R,5R,8S)-4,8-dimethyl-7-oxo -2,3-dioxabicyclo[3.3.1]nonan-4-yl]vinyl]benzonitril [Smp. 155 ° ];

gg) unter Verwendung eines entsprechenden aus (5R)-(-)-Carvon hergestellten Ausgansstoffes und des mittels Pentafluorbenzyltriphenylphosphoniumbromid und Natrium-bis(trimethylsilyl)amid hergestellten Pentaflurobenzylidentriphenylphosphoranes das (1S,4R,5R,8S)-4,8-Dimethyl-4-[(E)-2,3,4,5,6-pentafluorstyryl]-2,3-dioxabicyclo[3.3.1]nonan-7-on [Smp. 128 ° ];

hh) unter Verwendung des mittels 2,4-Dichlorbenzyltriphenylphosphoniumchlorid und Natrium-bis-(trimethylsilyl)amid in situ hergestellten 2,4-Dichlorbenzylidentriphenylphosphoranes das (1R,4S,5S,8R)-4-[(Z)-2,4-Dichlorstyryl]-4,8-dimethyl-2,3 -dioxabicyclo[3.3.1]nonan-7-on [Smp. 137 ° ];

ii) unter Verwendung des mittels 2,4-Bis(trifluormethyl)benzyltriphenylphosponiumbromid und Natrium-bis(trimethylsilyl)amid in situ hergestellten 2,4-Bis(trifluormethyl)benzylidentriphenylphosphoranes das (1R,4S,5S,8R)-4-[(Z)-2,4-Bis(trifluormethyl)styryl]-4,8-dimethyl-2,3 -dioxabicyclo[3.3.1]nonan-7-on [Smp. 124 ° ];

jj) unter Verwendung des mittels 2-Chlor-4-(trifluormethyl)benzyltriphenylphosphoniumchlorid und Natrium-bis(trimethylsilyl)amid in situ hergestellten 2-Chlor-4-(trifluormethyl)-benzylidentriphenylphosphoranes das (1R,4S,5S,8R)-4-[(Z)-2-Chlor-4-(trifluormethyl)styryl]-4,8-dimethyl-

2,3 -dioxabicyclo[3.3.1]nonan-7-on [Smp. 160° ];
kk) unter Verwendung des mittels 3,5-Dicyanobenzyltriphenylphosphoniumbromid und Natrium-bis-(trimethylsilyl)amid in situ hergestellten 3,5-Dicyanobenzylidentriphenylphosphoranes das 5-[(Z)-2-(1R,4S,5S,8R)-4,8-Dimethyl-7-oxo-2,3-dioxabicyclo[3.3.1]nonan-4-yl]vinyl]isophthalonitril [Smp. 185° ];
ll) unter Verwendung des mittels Pentafluorbenzyltriphenylphosphoniumbromid und Natrium-bis-(trimethylsilyl)amid in situ hergestellten Pentaflurobenzylidentriphenylphosphoranes das (1R,4S,5S,8R)-4,8-Dimethyl-4-[(E)-2,3,4,5,6-pentafluorstyryl]-2,3-dioxabicyclo[3.3.1]nonan-7-on [Smp. 128° ].

Beispiel A

4,8-Dimethyl-4-undecyl-2,3-dioxabicyclo[3.3.1]nonan-7-on kann als Wirkstoff nach an sich bekannten Methoden zu pharmazeutischen Präparaten nachfolgender Zusammensetzung formuliert werden:

| 1. Tabletten à 500 mg | |
|---|---|
| Wirkstoff | 500 mg |
| Lactose pulv. | 149 mg |
| Polyvinylpyrrolidon | 15 mg |
| Dioctylnatriumsulfosuccinat | 1 mg |
| Na-carboxymethylstärke | 30 mg |
| Magnesiumstearat | 5 mg |
| | 700 mg |

| 2. Tabletten à 50 mg | |
|---|---|
| Wirkstoff | 50 mg |
| Lactose pulv. | 50 mg |
| Mikrokristalline Cellulose | 82 mg |
| Na-carboxymethylstärke | 15 mg |
| Magnesiumstearat | 3 mg |
| | 200 mg |

| 3. Kapseln à 100 mg | |
|---|---|
| Wirkstoff | 100,0 mg |
| Lactose pulv. | 104,7 mg |
| Maisstärke | 70,0 mg |
| Hydroxypropylmethylcellulose | 10,0 mg |
| Dioctylnatriumsulfosuccinat | 0,3 mg |
| Talk | 12,0 mg |
| Magnesiumstearat | 3,0 mg |
| | 300,0 mg |

| 4. Suppositorien à 500 mg | |
|---|---|
| Wirkstoff | 500 mg |
| Suppositorienmasse | ad 2000 mg |

| 5. Weichgelatinekapsel à 100 mg | |
|---|---|
| Wirkstoff | 100 mg |
| Triglycerid mittelkettig | 300 mg |
| | 400 mg |

## Patentansprüche

## Patentansprüche für folgende Vertragsstaaten : AT, BE, CH, DE, FR, GB, IT, LI, LU, NL, SE

**1.** Verbindungen der allgemeinen Formel

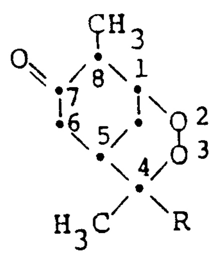

I

worin R eine gesättigte oder partiell ungesättigte Kohlenwasserstoffgruppe mit bis zu 15 Kohlenstoffatomen oder eine über eine Alkyl- oder Alkenylgruppe mit bis zu 7 Kohlenstoffatomen gebundene Aryl-, Heteroaryl-, Arylcarbonyl- oder Heteroarylcarbonylgruppe bedeutet,
wobei Aryl und Heteroaryl in den zuvor genannten Gruppen unsubstituiert oder mit bis zu 5 gleichen oder verschiedenen Substituenten ausgewählt aus Alkyl oder Alkoxy mit jeweils höchstens 7 Kohlenstoffatomen, Halogen, Trifluormethyl, Phenyl und Cyano substituiert sind.

**2.** Verbindungen nach Anspruch 1 in Form der (1R,4R,5S,8R)-, (1R,4S,5S,8R)-, (1S,4R,5R,8S)- oder der (1S,4S,5R,8S)-Isomeren oder in Form von Mischungen davon.

**3.** Verbindungen nach Anspruch 1 oder 2, worin R eine gesättigte Kohlenwasserstoffgruppe mit 8 bis 12 Kohlenstoffatomen bedeutet.

**4.** Verbindungen nach Anspruch 3, worin die Kohlenwasserstoffgruppe offenkettig und geradkettig ist.

**5.** 4,8-Dimethyl-4-octyl-2,3-dioxabicyclo[3.3.1]nonan-7-on.

**6.** 4,8-Dimethyl-4-nonyl-2,3-dioxabicyclo[3.3.1]nonan-7-on.

**7.** 4-Decyl-4,8-dimethyl-2,3-dioxabicyclo[3.3.1]nonan-7-on.

**8.** 4,8-Dimethyl-4-undecyl-2,3-dioxabicyclo[3.3.1]nonan-7-on.

**9.** 4-Dodecyl-4,8-dimethyl-2,3-dioxabicyclo[3.3.1]nonan-7-on.

**10.** Verbindungen nach Anspruch 1 oder 2, worin R eine über eine Alkenylgruppe mit bis zu 7 Kohlenstoffatomen gebundene Aryl- oder Heteroarylgruppe bedeutet.

**11.** Verbindungen nach Anspruch 10, worin die Arylgruppe eine durch Halogen und/oder Trifluormethyl mono-, di- oder pentasubstituierte Phenylgruppe und die Heteroarylgruppe eine durch Halogen und/oder Trifluormethyl mono-, di- oder trisubstituierte Chinolinylgruppe ist.

**12.** 4-[(Z)-2-[2,7-Bis(trifluormethyl)-4-chinolinyl]vinyl]-4,8-dimethyl-2,3-dioxabicyclo[3.3.1]nonan-7-on.

**13.** 4-[(Z)-2,4-Dichlorstyryl]-4,8-dimethyl-2,3-dioxabicyclo[3.3.1]nonan-7-on.

**14.** 4-[(Z)-2,4-Bis(trifluormethyl)styryl]-4,8-dimethyl-2,3-dioxabicyclo[3.3.1]nonan-7-on.

**15.** 4,8-Dimethyl-4-[(E)-2,3,4,5,6-pentafluorstyryl]-2,3-dioxabicyclo[3.3.1]nonan-7-on.

**16.** Verbindungen der allgemeinen Formeln

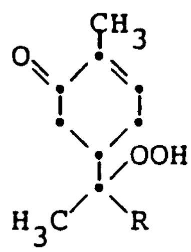

II

und

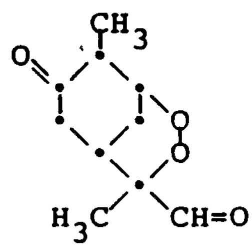

III

worin R eine gesättigte oder partiell ungesättigte Kohlenwasserstoffgruppe mit bis zu 15 Kohlenstoffatomen oder eine über eine Alkyl- oder Alkenylgruppe mit bis zu 7 Kohlenstoffatomen gebundene Aryl-, Heteroaryl-, Arylcarbonyl- oder Heteroarylcarbonylgruppe bedeutet,
wobei Aryl und Heteroaryl in den zuvor genannten Gruppen unsubstituiert oder mit bis zu 5 gleichen oder verschiedenen Substituenten ausgewählt aus Alkyl oder Alkoxy mit jeweils höchstens 7 Kohlenstoffatomen, Halogen, Trifluormethyl, Phenyl und Cyano substituiert sind.

**17.** Verbindungen nach einem der Ansprüche 1-15 zur Anwendung als therapeutische Wirkstoffe.

**18.** Verbindungen nach einem der Ansprüche 1-15 zur Anwendung als Wirkstoffe zur Verhütung oder Behandlung der Malaria.

**19.** Verfahren zur Herstellung von Verbindungen der allgemeinen Formel

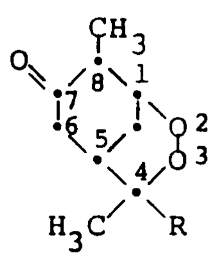

I

worin R eine gesättigte oder partiell ungesättigte Kohlenwasserstoffgruppe mit bis zu 15 Kohlenstoffatomen oder eine über eine Alkyl- oder Alkenylgruppe mit bis zu 7 Kohlenstoffatomen gebundene Aryl-, Heteroaryl-, Arylcarbonyl- oder Heteroarylcarbonylgruppe bedeutet,
wobei Aryl und Heteroaryl in den zuvor genannten Gruppen unsubstituiert oder mit bis zu 5 gleichen oder verschiedenen Substituenten ausgewählt aus Alkyl oder Alkoxy mit jeweils höchstens 7 Kohlenstoffatomen, Halogen, Trifluormethyl, Phenyl und Cyano substituiert sind,
dadurch gekennzeichnet, dass man

a) eine Verbindung der allgemeinen Formel

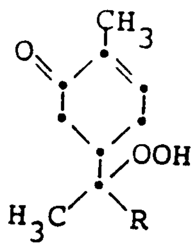

II

worin R obige Bedeutung besitzt,
in Gegenwart einer Base oder einer Säure cyclisiert, oder
b) eine Verbindung der Formel

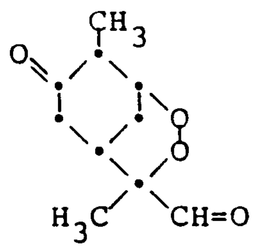

III

mit einem Phosphoran der allgemeinem Formel

Ø$_3$P = CH-R' IV

worin Ø Phenyl und R' Wasserstoff, eine gesättigte oder partiell ungesättigte Kohlenwasserstoffgruppe mit bis zu 13 Kohlenstoffatomen oder eine gegebenenfalls über eine Alkyl- oder Alkenylgruppe mit bis zu 5 Kohlenstoffatomen gebundene Aryl-, Heteroaryl-, Arylcarbonyl- oder Heteroarylcarbonylgruppe bedeutet, wobei Aryl und Heteroaryl in den zuvor genannten Gruppen unsubstituiert oder mit bis zu 5 gleichen oder verschiedenen Substituenten ausgewählt aus Alkyl oder Alkoxy mit jeweils höchstens 7 Kohlenstoffatomen, Halogen, Trifluormethyl, Phenyl und Cyano substituiert sind, umsetzt, oder
c) die Doppelbindung in einer Verbindung der allgemeinen Formel

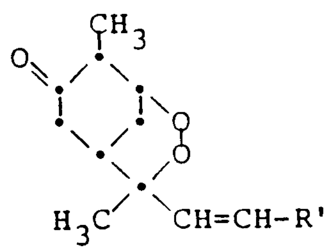

Ia

worin R' obige Bedeutung besitzt,
reduziert.

**20.** Arzneimittel enthaltend eine Verbindung nach einem der Ansprüche 1-15 und ein therapeutisch inertes Trägermaterial.

**21.** Mittel zur Verhütung oder Behandlung der Malaria enthaltend eine Verbindung nach einem der Ansprüche 1-15 und ein therapeutisch inertes Trägermaterial.

22. Verwendung von Verbindungen nach einem der Ansprüche 1-15 zur Herstellung von Mitteln zur Verhütung oder Behandlung der Malaria.

**Patentansprüche für folgende Vertragsstaaten : ES, GR**

1. Verfahren zur Herstellung von Verbindungen der allgemeinen Formel

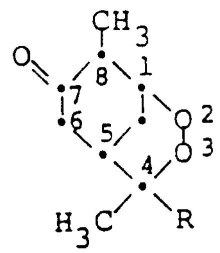

I

worin R eine gesättigte oder partiell ungesättigte Kohlenwasserstoffgruppe mit bis zu 15 Kohlenstoffatomen oder eine über eine Alkyl- oder Alkenylgruppe mit bis zu 7 Kohlenstoffatomen gebundene Aryl-, Heteroaryl-, Arylcarbonyl- oder Heteroarylcarbonylgruppe bedeutet,
wobei Aryl und Heteroaryl in den zuvor genannten Gruppen unsubstituiert oder mit bis zu 5 gleichen oder verschiedenen Substituenten ausgewählt aus Alkyl oder Alkoxy mit jeweils höchstens 7 Kohlenstoffatomen, Halogen, Trifluormethyl, Phenyl und Cyano substituiert sind,
dadurch gekennzeichnet, dass man
a) eine Verbindung der allgemeinen Formel

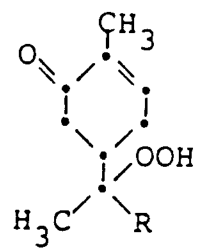

II

worin R obige Bedeutung besitzt,
in Gegenwart einer Base oder einer Säure cyclisiert, oder
b) eine Verbindung der Formel

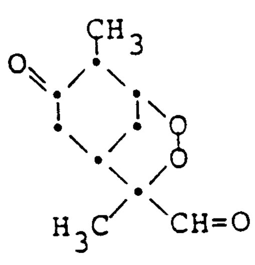

III

mit einem Phosphoran der allgemeinen Formel

$Ø_3P=CH\text{-}R'$ IV

worin Ø Phenyl und R' Wasserstoff, eine gesättigte oder partiell ungesättigte Kohlenwasserstoffgruppe mit bis zu 13 Kohlenstoffatomen oder eine gegebenenfalls über eine Alkyl- oder Alkenylgruppe mit bis zu 5 Kohlenstoffatomen gebundene Aryl-, Heteroaryl-, Arylcarbonyl- oder Heteroarylcarbonylgruppe bedeutet, wobei Aryl und Heteroaryl in den zuvor genannten Gruppen unsubstituiert oder

mit bis zu 5 gleichen oder verschiedenen Substituenten ausgewählt aus Alkyl oder Alkoxy mit jeweils höchstens 7 Kohlenstoffatomen, Halogen, Trifluormethyl, Phenyl und Cyano substituiert sind, umsetzt, oder

c) die Doppelbindung in einer Verbindung der allgemeinen Formel

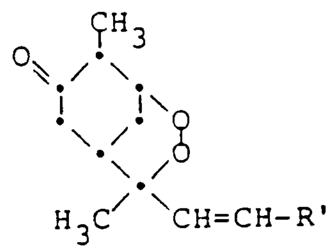

Ia

worin R' obige Bedeutung besitzt,

reduziert.

**2.** Verfahren nach Anspruch 1, dadurch gekennzeichnet, dass man die Verbindung der Formel I in Form des (1R,4R,5S,8R)-, (1R,4S,5S,8R)-, (1S,4R,5R,8S)- oder des (1S,4S,5R,8S)-Isomeren oder in Form einer Mischung davon herstellt.

**3.** Verfahren nach Anspruch 1 oder 2, worin R eine gesättigte Kohlenwasserstoffgruppe mit 8 bis 12 Kohlenstoffatomen bedeutet.

**4.** Verfahren nach Anspruch 3, worin die Kohlenwasserstoffgruppe offenkettig und geradkettig ist.

**5.** Verfahren nach Anspruch 1, dadurch gekennzeichnet, dass man 4,8-Dimethyl-4-octyl-2,3-dioxabicyclo-[3.3.1]nonan-7-on herstellt.

**6.** Verfahren nach Anspruch 1, dadurch gekennzeichnet, dass man 4,8-Dimethyl-4-nonyl-2,3-dioxabicyclo-[3.3.1]nonan-7-on herstellt.

**7.** Verfahren nach Anspruch 1, dadurch gekennzeichnet, dass man 4-Decyl-4,8-dimethyl-2,3-dioxabicyclo-[3.3.1]nonan-7-on herstellt.

**8.** Verfahren nach Anspruch 1, dadurch gekennzeichnet, dass man 4,8-Dimethyl-4-undecyl-2,3-dioxabicyclo[3.3.1]nonan-7-on herstellt.

**9.** Verfahren nach Anspruch 1, dadurch gekennzeichnet, dass man 4-Dodecyl-4,8-dimethyl-2,3-dioxabicyclo[3.3.1]nonan-7-on herstellt.

**10.** Verfahren nach Anspruch 1 oder 2, worin R eine über eine Alkenylgruppe mit bis zu 7 Kohlenstoffatomen gebundene Aryl- oder Heteroarylgruppe bedeutet.

**11.** Verfahren nach Anspruch 10, worin die Arylgruppe eine durch Halogen und/oder Trifluormethyl mono-, di- oder pentasubstituierte Phenylgruppe und die Heteroarylgruppe eine durch Halogen und/oder Trifluormethyl mono-, di- oder trisubstituierte Chinolinylgruppe ist.

**12.** Verfahren nach Anspruch 1, dadurch gekennzeichnet, dass man 4-[(Z)-2-[2,7-Bis(trifluormethyl)-4-chinolinyl]vinyl]-4,8-dimethyl-2,3-dioxabicyclo[3.3.1]nonan-7-on herstellt.

**13.** Verfahren nach Anspruch 1, dadurch gekennzeichnet, dass man 4-[(Z)-2,4-Dichlorstyryl]-4,8-dimethyl-2,3-dioxabicyclo[3.3.1]nonan-7-on herstellt.

**14.** Verfahren nach Anspruch 1, dadurch gekennzeichnet, dass man 4-[(Z)-2,4-Bis(trifluormethyl)styryl]-4,8-dimethyl-2,3-dioxabicyclo[3.3.1]nonan-7-on herstellt.

15. Verfahren nach Anspruch 1, dadurch gekennzeichnet, dass man 4,8-Dimethyl-4-[(E)-2,3,4,5,6-pentafluorstyryl]-2,3-dioxabicyclo[3.3.1]nonan-7-on herstellt.

16. Verfahren zur Herstellung von Mitteln, insbesondere von Mitteln zur Verhütung oder Behandlung der Malaria, dadurch gekennzeichnet, dass man eine Verbindung der in Anspruch 1 definierten Formel I und gegebenenfalls andere therapeutisch wertvolle Stoffe zusammen mit einem therapeutisch inerten Trägermaterial in eine galenische Darreichungsform bringt.

17. Verwendung von Verbindungen der in Anspruch 1 definierten Formel I zur Herstellung von Mitteln zur Verhütung oder Behandlung der Malaria.

18. Verbindungen der allgemeinen Formeln

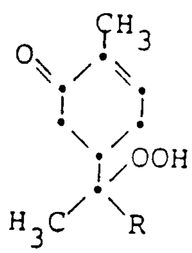

II

und

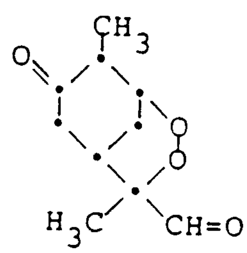

III

worin R eine gesättigte oder partiell ungesättigte Kohlenwasserstoffgruppe mit bis zu 15 Kohlenstoffatomen oder eine über eine Alkyl- oder Alkenylgruppe mit bis zu 7 Kohlenstoffatomen gebundene Aryl-, Heteroaryl-, Arylcarbonyl- oder Heteroarylcarbonylgruppe bedeutet,
wobei Aryl und Heteroaryl in den zuvor genannten Gruppen unsubstituiert oder mit bis zu 5 gleichen oder verschiedenen Substituenten ausgewählt aus Alkyl oder Alkoxy mit jeweils höchstens 7 Kohlenstoffatomen, Halogen, Trifluormethyl, Phenyl und Cyano substituiert sind.

**Claims**

**Claims for the following Contracting States : AT, BE, CH, DE, FR, GB, IT, LI, LU, NE, SE**

1. Compounds of the general formula

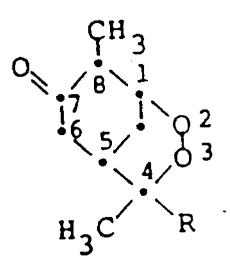

I

wherein R signifies a saturated or partially unsaturated hydrocarbon group with up to 15 carbon atoms or an aryl, heteroaryl, arylcarbonyl or heteroarylcarbonyl group attached via an alkyl or alkenyl group with up to 7 carbon atoms, whereby aryl and heteroaryl in the aforementioned groups are unsubstituted or are substituted with up to 5 identical or different substituents selected from alkyl or alkoxy each with a maximum of 7 carbon atoms, halogen, trifluoromethyl, phenyl and cyano.

2. Compounds according to claim 1 in the form of the (1R,4R,5S,8R)-, (1R,4S,5S,8R)-, (1S,4R,5R,8S)- or the (1S,4S,5R,8S)-isomers or in the form of mixtures thereof.

3. Compounds according to claim 1 or 2, wherein R signifies a saturated hydrocarbon group with 8 to 12 carbon atoms.

4. Compounds according to claim 3, wherein the hydrocarbon group is open-chain and straight-chain.

**5.** 4,8-Dimethyl-4-octyl-2,3-dioxabicyclo[3.3.1]nonan-7-one.

**6.** 4,8-Dimethyl-4-nonyl-2,3-dioxabicyclo[3.3.1]nonan-7-one.

**7.** 4-Decyl-4,8-dimethyl-2,3-dioxabicyclo[3.3.1]nonan-7-one.

**8.** 4,8-Dimethyl-4-undecyl-2,3-dioxabicyclo[3.3.1]nonan-7-one.

**9.** 4-Dodecyl-4,8-dimethyl-2,3-dioxabicyclo[3.3.1]nonan-7-one.

**10.** Compounds according to claim 1 or 2, wherein R signifies an aryl or heteroaryl group attached via an alkenyl group with up to 7 carbon atoms.

**11.** Compounds according to claim 10, wherein the aryl group is a phenyl group mono-, di- or pentasubstituted by halogen and/or trifluoromethyl and the heteroaryl group is a quinolinyl group mono-, di- or trisubstituted by halogen and/or trifluoromethyl.

**12.** 4-[(Z)-2-[2,7-Bis(trifluoromethyl-4-quinolinyl]vinyl]-4,8-dimethyl-2,3-dioxabicyclo[3.3.1]nonan-7-one.

**13.** 4-[(Z)-2,4-Dichlorostyryl]-4,8-dimethyl-2,3-dioxabicyclo[3.3.1]nonan-7-one.

**14.** 4-[(Z)-2,4-Bis(trifluoromethyl)styryl]-4,8-dimethyl-2,3-dioxabicyclo[3.3.1]nonan-7-one.

**15.** 4,8-Dimethyl-4-[(E)-2,3,4,5,6-pentafluorostyryl]-2,3-dioxabicyclo[3.3.1]nonan-7-one.

**16.** Compounds of the general formulae

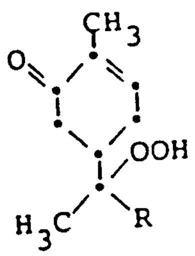

II

and

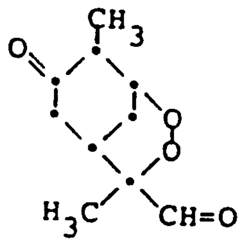

III

wherein R signifies a saturated or partially unsaturated hydrocarbon group with up to 15 carbon atoms or an aryl, heteroaryl, arylcarbonyl or heteroarylcarbonyl group attached via an alkyl or alkenyl group with up to 7 carbon atoms, whereby aryl and heteroaryl in the aforementioned groups are unsubstituted or are substituted with up to 5 identical or different substituents selected from alkyl or alkoxy each with a maximum of 7 carbon atoms, halogen, trifluoromethyl, phenyl and cyano.

**17.** Compounds according to any one of claims 1-15 for use as therapeutically active substances.

**18.** Compounds according to any one of claims 1-15 for use as active substances for the prevention or treatment of malaria.

**19.** A process for the manufacture of compounds of the general formula

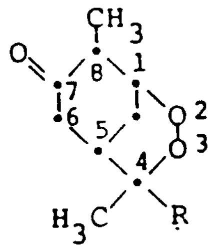

I

wherein R signifies a saturated or partially unsaturated hydrocarbon group with up to 15 carbon atoms or an aryl, heteroaryl, arylcarbonyl or heteroarylcarbonyl group attached via an alkyl or alkenyl group with up to 7 carbon atoms, whereby aryl and heteroaryl in the aforementioned groups are unsubstituted or are substituted with up to 5 identical or different substituents selected from alkyl or alkoxy each with a maximum of 7 carbon atoms, halogen, trifluoromethyl, phenyl and cyano,
characterized by

a) cyclizing a compound of the general formula

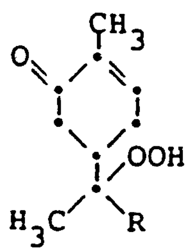

II

wherein R has the above significance,
in the presence of a base or of an acid, or

b) reacting a compound of the formula

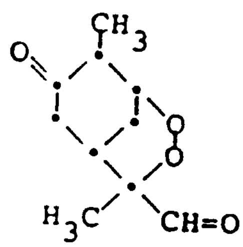

III

with a phosphorane of the general formula

$Ø_3P=CH\text{-}R'$ IV

wherein Ø signifies phenyl and R' signifies hydrogen, a saturated or partially unsaturated hydrocarbon group with up to 13 carbon atoms or an aryl, heteroaryl, arylcarbonyl or heteroarylcarbonyl group attached via an alkyl or alkenyl group with up to 5 carbon atoms, whereby aryl and heteroaryl in the aforementioned groups are unsubstituted or are substituted with up to 5 identical or different substituents selected from alkyl or alkoxy each with a maximum of 7 carbon atoms, halogen, trifluoromethyl, phenyl and cyano,
or

c) reducing the double bond in a compound of the general formula

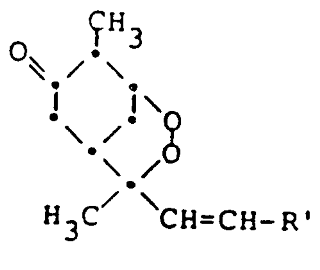

Ia

wherein R' has the above significance.

**20.** A medicament containing a compound according to any one of claims 1-15 and a therapeutically inert carrier material.

**21.** A medicament for the prevention or treatment of malaria, containing a compound according to any one of claims 1-15 and a therapeutically inert carrier material.

**22.** The use of compounds according to any one of claims 1-15 for the manufacture of medicaments for the prevention or treatment of malaria.

**Claims for the following Contracting States : ES, GR**

**1.** A process for the manufacture of compounds of the general formula

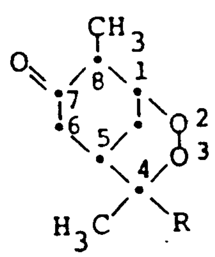

I

wherein R signifies a saturated or partially unsaturated hydrocarbon group with up to 15 carbon atoms or an aryl, heteroaryl, arylcarbonyl or heteroarylcarbonyl group attached via an alkyl or alkenyl group with up to 7 carbon atoms, whereby aryl and heteroaryl in the aforementioned groups are unsubstituted or are substituted with up to 5 identical or different substituents selected from alkyl or alkoxy each with a maximum of 7 carbon atoms, halogen, trifluoromethyl, phenyl and cyano,
characterized by

a) cyclizing a compound of the general formula

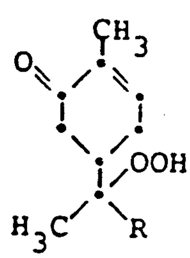

II

wherein R has the above significance,

in the presence of a base or of an acid, or
b) reacting a compound of the formula

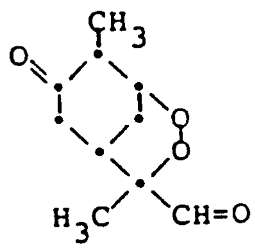

III

with a phosphorane of the general formula

$Ø_3P=CH\text{-}R'$ IV

wherein Ø signifies phenyl and R' signifies hydrogen, a saturated or partially unsaturated hydrocarbon group with up to 13 carbon atoms or an aryl, heteroaryl, arylcarbonyl or heteroarylcarbonyl group attached via an alkyl or alkenyl group with up to 5 carbon atoms, whereby aryl and heteroaryl in the aforementioned groups are unsubstituted or are substituted with up to 5 identical or different substituents selected from alkyl or alkoxy each with a maximum of 7 carbon atoms, halogen, trifluoromethyl, phenyl and cyano,
or
c) reducing the double bond in a compound of the general formula

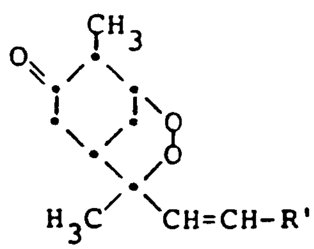

Ia

wherein R' has the above significance.

2. A process according to claim 1, characterized in that the compound of formula I is manufactured in the form of the (1R,4R,5S,8R)-, (1R,4S,5S,8R)-, (1S,4R,5R,8S)- or the (1S,4S,5R,8S)-isomers or in the form of mixtures thereof.

3. A process according to claim 1 or 2, wherein R signifies a saturated hydrocarbon group with 8 to 12 carbon atoms.

4. A process according to claim 3, wherein the hydrocarbon group is open-chain and straight-chain.

5. A process according to claim 1, characterized in that 4,8-dimethyl-4-octyl-2,3-dioxabicyclo[3.3.1]nonan-7-one is manufactured.

6. A process according to claim 1, characterized in that 4,8-dimethyl-4-nonyl-2,3-dioxabicyclo[3.3.1]nonan-7-one is manufactured.

7. A process according to claim 1, characterized in that 4-decyl-4,8-dimethyl-2,3-dioxabicyclo[3.3.1]nonan-7-one is manufactured.

**8.** A process according to claim 1, characterized in that 4,8-dimethyl-4-undecyl-2,3-dioxabicyclo[3.3.1]-nonan-7-one is manufactured.

**9.** A process according to claim 1, characterized in that 4-dodecyl-4,8-dimethyl-2,3-dioxabicyclo[3.3.1]-nonan-7-one is manufactured.

**10.** A process according to claim 1 or 2, wherein R signifies an aryl or heteroaryl group attached via an alkenyl group with up to 7 carbon atoms.

**11.** A process according to claim 10, wherein the aryl group is a phenyl group mono-, di- or pentasubstituted by halogen and/or trifluoromethyl and the heteroaryl group is a quinolinyl group mono-, di- or trisubstituted by halogen and/or trifluoromethyl.

**12.** A process according to claim 1, characterized in that 4-[(Z)-2-[2,7-bis(trifluoromethyl-4-quinolinyl]vinyl]-4,8-dimethyl-2,3-dioxabicyclo[3.3.1]nonan-7-one is manufactured.

**13.** A process according to claim 1, characterized in that 4-[(Z)-2,4-dichlorostyryl]-4,8-dimethyl-2,3-dioxabicyclo[3.3.1]nonan-7-one is manufactured.

**14.** A process according to claim 1, characterized in that 4-[(Z)-2,4-bis(trifluoromethyl)styryl]-4,8-dimethyl-2,3-dioxabicyclo[3.3.1]nonan-7-one is manufactured.

**15.** A process according to claim 1, characterized in that 4,8-dimethyl-4-[(E)-2,3,4,5,6-pentafluorostyryl]-2,3-dioxabicyclo[3.3.1]nonan-7-one is manufactured.

**16.** A process for the manufacture of medicaments, especially of medicaments for the prevention or treatment of maleria, characterized by bringing a compound of formula I defined in claim 1 and, if desired, other therapeutically valuable substances into a galenical administration form together with a therapeutically inert carrier.

**17.** The use of compounds of formula I defined in claim 1 for the manufacture of medicaments for the prevention or treatment of malaria.

**18.** Compounds of the general formulae

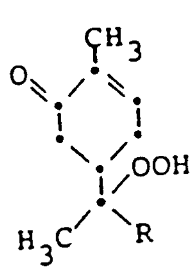

II

and

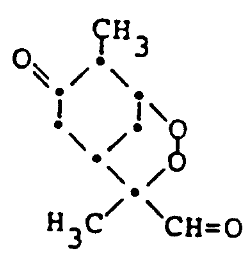

III

wherein R signifies a saturated or partially unsaturated hydrocarbon group with up to 15 carbon atoms or an aryl, heteroaryl, arylcarbonyl or heteroarylcarbonyl group attached via an alkyl or alkenyl group with up to 7 carbon atoms, whereby aryl and heteroaryl in the aforementioned groups are unsubstituted or are substituted with up to 5 identical or different substituents selected from alkyl or alkoxy each with a maximum of 7 carbon atoms, halogen, trifluoromethyl, phenyl and cyano.

**Revendications**

**Revendications pour les Etats contractants suivants : AT, BE, CH, DE, FR, GB, IT, LI, LU, NL, SE**

1. Composés de formule générale

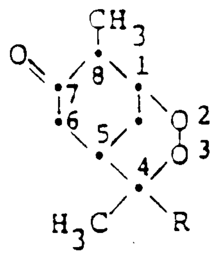

I

dans laquelle R est un groupe hydrocarbure saturé ou partiellement insaturé ayant jusqu'à 15 atomes de carbone ou un groupe hétéroarylcarbonyle, arylcarbonyle, hétéroaryle ou aryle fixé par l'intermédiaire d'un groupe alkyle ou alcényle ayant jusqu'à 7 atomes de carbone, les aryles et hétéroaryles dans les groupes cités plus haut étant non substitués ou substitués avec jusqu'à cinq substituants identiques ou différents choisis parmi les alkyle ou alcoxy, chacun ayant au plus 7 atomes de carbone, halogène, trifluorométhyle, phényle et cyano.

2. Composés selon la revendication 1 sous forme des isomères (1R,4R,5S,8R)-, (1R,4S,5S,8R)-, (1S,4R,5R,8S)- ou (1S,4S,5R,8S) ou sous forme de leurs mélanges.

3. Composés selon la revendication 1 ou 2, dans lesquels R est un groupe hydrocarbure saturé avec 8 à 12 atomes de carbone.

4. Composés selon la revendication 3, dans lesquels le groupe hydrocarbure est à chaîne droite et ouverte.

5. 4,8-diméthyl-4-octyl-2,3-dioxabicyclo[3.3.1]nonan-7-one.

6. 4,8-diméthyl-4-nonyl-2,3-dioxabicyclo[3.3.1]nonan-7-one.

7. 4-décyl-4,8-diméthyl-2,3-dioxabicyclo[3.3.1]nonan-7-one.

8. 4,8-diméthyl-4-undécyl-2,3-dioxabicyclo[3.3.1]nonan-7-one.

9. 4-dodécyl-4,8-diméthyl-2,3-dioxabicyclo[3.3.1]nonan-7-one.

10. Composés selon la revendication 1 ou 2, dans lesquels R est un groupe aryle ou hétéroaryle fixé par un groupe alcényle ayant jusqu'à 7 atomes de carbone.

11. Composés selon la revendication 10, dans lesquels le groupe aryle est un groupe phényle mono-, di- ou penta-substitué et le groupe hétéroaryle est un groupe chinolyle mono-, di- ou trisubstitué par halogène et/ou trifluorométhyle.

12. 4-[(Z)-2-[2,7-bis(trifluorométhyl)-4-chinolinyl]-vinyl]-4,8-diméthyl-2,3-dioxabicyclo[3.3.1]nonan-7-one.

13. 4-[(Z)-2,4-dichlorostyryl]-4,8-diméthyl-2,3-dioxabicyclo[3.3.1]nonan-7-one.

14. 4-[(Z)-2,4-bis(trifluorométhyl)styryl]-4,8-diméthyl-2,3-dioxabicyclo[3.3.1]nonan-7-one.

15. 4,8-diméthyl-4-[(E)-2,3,4,5,6-pentafluorostyryl]-2,3-dioxabicyclo[3.3.1]nonan-7-one.

16. Composés de formules générales

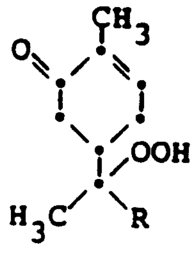

II et

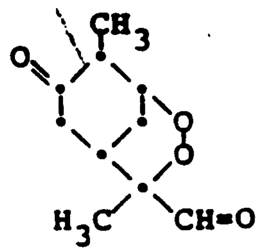

III

dans lesquelles R est un groupe hydrocarbure saturé ou partiellement insaturé ayant jusqu'à 15 atomes de carbone ou un groupe hétéroarylcarbonyle, arylcarbonyle, hétéroaryle ou aryle fixé par l'intermédiaire d'un groupe alkyle ou alcényle ayant jusqu'à 7 atomes de carbone, les aryles et hétéroaryles dans les groupes cités plus haut étant non substitués ou substitués avec jusqu'à cinq substituants identiques ou différents choisis parmi les alkyle ou alcoxy, chacun ayant au plus 7 atomes de carbone, halogène, trifluorométhyle, phényle et cyano.

17. Composés selon l'une des revendications 1-15 pour l'utilisation comme substances thérapeutiquement actives.

18. Composés selon l'une des revendications 1-15 pour l'utilisation comme principes actifs de prévention ou de traitement de la malaria.

19. Procédé de préparation de composés de formule générale

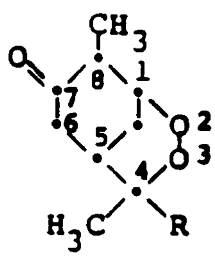

I

dans laquelle R est un groupe hydrocarbure saturé ou partiellement insaturé ayant jusqu'à 15 atomes de carbone ou un groupe hétéroarylcarbonyle, arylcarbonyle, hétéroaryle ou aryle fixé par l'intermédiaire d'un groupe alkyle ou alcényle ayant jusqu'à 7 atomes de carbone, les aryles et hétéroaryles dans les groupes cités plus haut étant non substitués ou substitués avec jusqu'à cinq substituants identiques ou différents choisis parmi les alkyle ou alcoxy, chacun ayant au plus 7 atomes de carbone, halogène, trifluorométhyle, phényle et cyano,
caractérisé en ce qu'on cyclise

a) un composé de formule générale

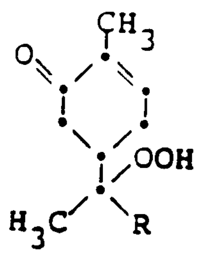

II

dans laquelle R a la signification ci-dessus, en présence d'un acide ou d'une base, ou
b) on fait réagir un composé de formule

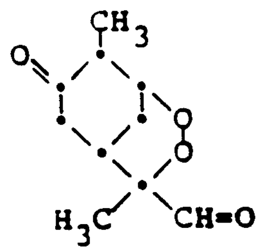

III

avec un phosphorane de formule générale

$Ø_3P=CH\text{-}R'$ IV

dans laquelle Ø est le phényle et R' l'hydrogène, un groupe hydrocarbure saturé ou partiellement insaturé ayant jusqu'à 13 atomes de carbone ou un groupe hétéroarylcarbonyle, arylcarbonyle, hétéroaryle ou aryle fixé le cas échéant par l'intermédiaire d'un groupe alkyle ou alcényle ayant jusqu'à 7 atomes de carbone, les aryles et hétéroaryles dans les groupes cités plus haut étant non substitués ou substitués avec jusqu'à cinq substituants identiques ou différents choisis parmi les alkyle ou alcoxy, chacun ayant au plus 7 atomes de carbone, halogène, trifluorométhyle, phényle et cyano, ou
c) on réduit la double liaison dans un composé de formule générale

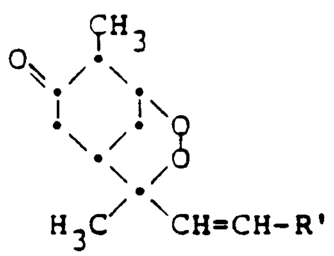

Ia

dans laquelle R' a la signification ci-dessus.

**20.** Médicament comprenant un composé selon l'une des revendications 1-15 et un matériau véhicule thérapeutiquement inerte.

**21.** Moyen de prévention ou de traitement de la malaria comprenant un composé selon l'une des revendications 1-15 et un matériau véhicule thérapeutiquement inerte.

**22.** Utilisation de composés selon l'une des revendications 1-15 pour préparer des moyens de prévention et de traitement de la malaria.

**Revendications pour les Etats contractants suivants : ES, GR**

**1.** Procédé de préparation de composés de formule générale

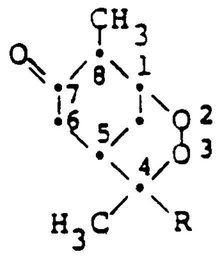

I

dans laquelle R est un groupe hydrocarbure saturé ou partiellement insaturé ayant jusqu'à 15 atomes de carbone ou un groupe hétéroarylcarbonyle, arylcarbonyle, hétéroaryle ou aryle fixé par l'intermédiaire d'un groupe alkyle ou alcényle ayant jusqu'à 7 atomes de carbone, les aryles et hétéroaryles dans les groupes cités plus haut étant non substitués ou substitués avec jusqu'à cinq substituants identiques ou différents choisis parmi les alkyle ou alcoxy, chacun ayant au plus 7 atomes de carbone, halogène, trifluorométhyle, phényle et cyano,
caractérisé en ce qu'on cyclise

a) un composé de formule générale

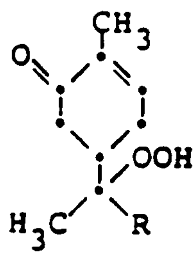

II

dans laquelle R a la signification ci-dessus,
en présence d'un acide ou d'une base, ou

b) on fait réagir un composé de formule

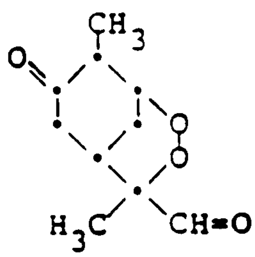

III

avec un phosphorane de formule générale

$Ø_3P=CH-R'$ IV

dans laquelle Ø est le phényle et R' l'hydrogène, un groupe hydrocarbure saturé ou partiellement insaturé ayant jusqu'à 13 atomes de carbone ou un groupe hétéroarylcarbonyle, arylcarbonyle, hétéroaryle ou aryle fixé le cas échéant par l'intermédiaire d'un groupe alkyle ou alcényle ayant jusqu'à 7 atomes de carbone, les aryles et hétéroaryles dans les groupes cités plus haut étant non

substitués ou substitués avec jusqu'à cinq substituants identiques ou différents choisis parmi les alkyle ou alcoxy, chacun ayant au plus 7 atomes de carbone, halogène, trifluorométhyle, phényle et cyano, ou

c) on réduit la double liaison dans un composé de formule générale

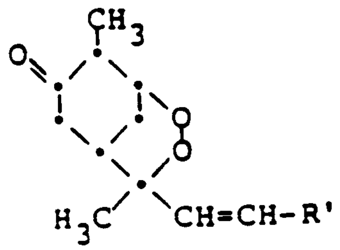

Ia

dans laquelle R' a la signification ci-dessus.

2. Procédé selon la revendication 1, caractérisé en ce qu'on prépare le composé de formule I sous forme des isomères (1R,4R,5S,8R)-, (1R,4S,5S,8R)-, (1S,4R,5R,8S)- ou (1S,4S,5R,8S) ou sous forme d'un de leurs mélanges.

3. Procédé selon la revendication 1 ou 2, dans lequel R est un groupe hydrocarbure saturé avec 8 à 12 atomes de carbone.

4. Procédé selon la revendication 3, dans lequel le groupe hydrocarbure est à chaîne droite et ouverte.

5. Procédé selon la revendication 1, caractérisé en ce qu'on prépare la 4,8-diméthyl-4-octyl-2,3-dioxabicyclo[3.3.1]nonan-7-one.

6. Procédé selon la revendication 1, caractérisé en ce qu'on prépare la 4,8-diméthyl-4-nonyl-2,3-dioxabicyclo[3.3.1]nonan-7-one.

7. Procédé selon la revendication 1, caractérisé en ce qu'on prépare la 4-décyl-4,8-diméthyl-2,3-dioxabicyclo[3.3.1]nonan-7-one.

8. Procédé selon la revendication 1, caractérisé en ce qu'on prépare la 4,8-diméthyl-4-undécyl-2,3-dioxabicyclo[3.3.1]nonan-7-one.

9. Procédé selon la revendication 1, caractérisé en ce qu'on prépare la 4-dodécyl-4,8-diméthyl-2,3-dioxabicyclo[3.3.1]nonan-7-one.

10. Procédé selon la revendication 1 ou 2, dans lequel R est un groupe aryle ou hétéroaryle fixé par un groupe alcényle ayant jusqu'à 7 atomes de carbone.

11. Procédé selon la revendication 10, dans lequel le groupe aryle est un groupe phényle, mono-, di- ou pentasubstitué et le groupe hétéroaryle est un groupe chinolyle mono-, di- ou trisubstitué par halogène et/ou trifluorométhyle.

12. Procédé selon la revendication 1, caractérisé en ce qu'on prépare la 4-[(Z)-2-[2,7-bis(trifluorométhyl)-4-chinolinyl]-vinyl]-4,8-diméthyl-2,3-dioxa bicyclo[3.3.1]nonan-7-one.

13. Procédé selon la revendication 1, caractérisé en ce qu'on prépare la 4-[(Z)-2,4-dichlorostyryl]-4,8-diméthyl-2,3-dioxabicyclo[3.3.1]nonan-7-one.

14. Procédé selon la revendication 1, caractérisé en ce qu'on prépare la 4-[(Z)-2,4-bis(trifluorométhyl)-styryl]-4,8-diméthyl-2,3-dioxabicyclo[3.3.1]nonan-7-one.

**15.** Procédé selon la revendication 1, caractérisé en ce qu'on prépare la 4,8-diméthyl-4-[(E)-2,3,4,5,6-pentafluorostyryl]-2,3-dioxabicyclo[3.3.1]nonan-7-one.

**16.** Procédé de préparation de moyens, notamment de moyens de prévention et de traitement de la malaria, caractérisé en ce qu'on met sous forme d'administration galénique un composé de formule I défini à la revendication 1 et le cas échéant en présence d'autres substances thérapeutiquement actives, avec un matériau véhicule thérapeutiquement inerte.

**17.** Utilisation de composés de formule I définie à la revendication 1 pour préparer des moyens de prévention ou de traitement de la malaria.

**18.** Composés de formules générales

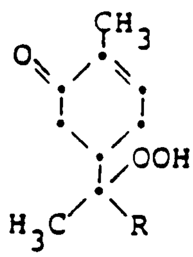

II et

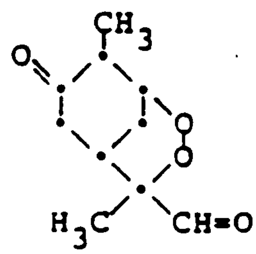

III

dans lesquelles R est un groupe hydrocarbure saturé ou partiellement insaturé ayant jusqu'à 15 atomes de carbone ou un groupe hétéroarylcarbonyle, arylcarbonyle, hétéroaryle ou aryle fixé par l'intermédiaire d'un groupe alkyle ou alcényle ayant jusqu'à 7 atomes de carbone, les aryles et hétéroaryles dans les groupes cités plus haut étant non substitués ou substitués avec jusqu'à cinq substituants identiques ou différents choisis parmi les alkyle ou alcoxy, chacun ayant au plus 7 atomes de carbone, halogène, trifluorométhyle, phényle et cyano.